(19) Europäisches Patentamt / European Patent Office / Office européen des brevets

(11) **EP 3 460 453 A2**

(12) **EUROPEAN PATENT APPLICATION**
published in accordance with Art. 153(4) EPC

(43) Date of publication:
**27.03.2019 Bulletin 2019/13**

(51) Int Cl.:
***G01N 21/359*** (2014.01)      ***G01N 21/31*** (2006.01)
***G01N 33/48*** (2006.01)

(21) Application number: **17831183.3**

(22) Date of filing: **17.02.2017**

(86) International application number:
**PCT/KR2017/001771**

(87) International publication number:
**WO 2018/016709 (25.01.2018 Gazette 2018/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **21.07.2016 KR 20160092845
04.01.2017 KR 20170001539**

(71) Applicant: **Infit&Company Inc.
Seoul 06729 (KR)**

(72) Inventors:
• **HAN, Sung Ho
Seoul 06649 (KR)**
• **NO, Keun Sik
Yongin-si
Gyeonggi-do 16989 (KR)**
• **HONG, Hee Sun
Gwacheon-si
Gyeonggi-do 13818 (KR)**

(74) Representative: **Manitz Finsterwald
Patent- und Rechtsanwaltspartnerschaft mbB
Martin-Greif-Strasse 1
80336 München (DE)**

(54) **FREQUENCY DOMAIN-BASED MULTI-WAVELENGTH BIOMETRIC SIGNAL ANALYSIS APPARATUS AND METHOD THEREOF**

(57)    The present disclosure discloses a frequency domain (FD)-based multi-wavelength bio-signal analyzing apparatus including: four or more light sources configured to irradiate frequency-modulated light at four or more different discrete wavelengths; at least one light detector configured to detect an output light reflected and introduced from a subject; and a processing circuit connected to the four or more light sources and the at least one light detector and configured to calculate a scattering coefficient and an absorption coefficient at each discrete wavelength based on an output light detected by the at least one light detector and calculate a concentration of a chromophore present in the subject based on the scattering coefficient and the absorption coefficient at each discrete wavelength. Herein, the processing circuit drives at least two of the four or more light sources based on the chromophore present in the subject.

*FIG. 1*

EP 3 460 453 A2

**Description**

**TECHNICAL FIELD**

The present disclosure relates to a frequency domain-based multi-wavelength bio-signal analyzing apparatus and a method thereof.

**BACKGROUND**

Recently, various techniques for analyzing biometric data of the body using a method for measuring the optical properties of a turbid medium are being developed. These techniques have attracted a lot of attention in that they are non-invasive and can provide biometric data, and a lot of attention is being focused on research for development into commercial devices according to the needs of consumers.

These techniques generally calculate the concentration of a chromophore in the turbid medium by measuring the absorption coefficient and the scattering coefficient of a turbid medium in a near infrared ray region. There are three methods known to measure the absorption coefficient and the scattering coefficient of a turbid medium. Specifically, these methods include a steady-state (SS) method of constantly irradiating light of a power of the same intensity during measurement into a turbid medium and calculating the concentration of a chromophore according to a multi-distance measurement method, a frequency domain (FD) method of measuring a changed amplitude and phase for a modulated light source, and a time domain (TD) method of measuring a change over time for a pulse-type light source.

However, the above-described three methods have their own merits and limitations.

The SS method does not require the modulation of light or pulse generation and thus does not require a detector that decomposes light reflected from a turbid medium by frequency domain or time domain. Therefore, the SS method is cheaper than the other methods (i.e., FD method or TD method). However, the SS method uses the multi-distance measurement method to separate the absorption coefficient and the scattering coefficient. Therefore, in biological tissue with high non-uniformity, the SS method is more likely to generate distortion during analysis than the other methods.

The TD method and the FD method do not use multi-distance measurement method and thus are more suitable for biological tissue with non-uniformity than the SS method. However, the TD method and the FD method require a detector configured to detect pulse generation or frequency-modulated light source and the properties thereof. Therefore, the TD method and the FD method have shortcomings in terms of implementation and cost.

U.S. Patent No. 7,428,434 (entitled "Quantitative Broadband Absorption and Scattering Spectroscopy in Turbid Media by Combined Frequency Domain and Steady State Methodologies") suggests a diffuse optical spectroscopic imaging method combining the FD method and the SS method. U.S. Patent No. 7,428,434 makes up for the shortcoming of the conventional FD and SS methods by combining a limited number of FD wavelengths and an SS method for distance measurement. However, a diffuse optical spectroscopic imaging apparatus combining the FD method and the SS method has limitations as an commercial apparatus in terms of apparatus size or cost.

**DISCLOSURE OF THE INVENTION**

**PROBLEMS TO BE SOLVED BY THE INVENTION**

There is a need for a bio-signal analyzing apparatus which is miniaturized and configured to measure a concentration value of a chromophore with high reliability using a limited number of light sources and enable a light source and a light detector to be in direct contact with a subject and thus can reduce cost.

**MEANS FOR SOLVING THE PROBLEMS**

The present disclosure provides an FD-based multi-wavelength bio-signal analyzing apparatus including: four or more light sources configured to irradiate frequency-modulated light at four or more different discrete wavelengths; at least one light detector configured to detect an output light reflected and introduced from a subject; and a processing circuit connected to the four or more light sources and the at least one light detector and configured to calculate a scattering coefficient and an absorption coefficient at each discrete wavelength based on an output light and calculate a concentration of a chromophore present in the subject based on the scattering coefficient and the absorption coefficient at each discrete wavelength.

**EFFECTS OF THE INVENTION**

According to an embodiment of the present disclosure, an FD-based multi-wavelength bio-signal analyzing apparatus uses a limited number of light sources. Thus, it is possible to provide a method and an apparatus capable of measuring a concentration value of a chromophore with high reliability at reduced implementation cost.

Further, according to an embodiment of the present disclosure, it is possible to provide a miniaturized FD-based multi-wavelength bio-signal analyzing apparatus and also possible to provide a bioassay result (i.e., concentration of a chromophore) corresponding to different depths and locations in a subject.

## BRIEF DESCRIPTION OF THE DRAWINGS

FIG. 1 is a diagram illustrating a configuration of a frequency domain (FD)-based multi-wavelength bio-signal analyzing apparatus according to an embodiment of the present disclosure.

FIG. 2 is a graph showing absorption spectra of chromophores present in the body.

FIG. 3 shows characteristic of an input light input into a subject and an output light detected by a light detector according to an embodiment of the present disclosure.

FIG. 4 is a diagram showing a process of calculating a concentration of each chromophore by the processing circuit illustrated in FIG. 1 according to an embodiment of the present disclosure.

FIG. 5 to FIG. 8 are graphs showing the result of comparison between an absorption coefficient spectrum obtained according to an embodiment of the present disclosure and an absorption coefficient spectrum which is reconstructed with a chromophore concentration obtained by fitting from an absorption coefficient obtained by an FD-SS (steady state) diffuse optical spectroscopic imaging apparatus.

FIG. 9 is a graph showing the result of $R^2$ analysis on a total of 72 data of the absorption coefficient spectra used in FIG. 5 to FIG. 8.

FIG. 10 to FIG. 13 are graphs showing concentration values of the respective chromophores calculated from the absorption coefficient spectra used in FIG. 5 to FIG. 8.

FIG. 14 is a diagram illustrating an FD-based multi-wavelength bio-signal analyzing apparatus according to an embodiment of the present disclosure.

FIG. 15 is a diagram illustrating an FD-based multi-wavelength bio-signal analyzing apparatus according to another embodiment of the present disclosure.

FIG. 16 is a diagram illustrating an FD-based multi-wavelength bio-signal analyzing apparatus according to yet another embodiment of the present disclosure.

FIG. 17 is a diagram illustrating a contact surface of the probe illustrated in FIG. 16.

FIG. 18 is another diagram illustrating a contact surface of the probe illustrated in FIG. 16.

FIG. 19 is a diagram illustrating an FD-based multi-wavelength bio-signal analyzing apparatus according to still another embodiment of the present disclosure.

FIG. 20 is a diagram illustrating an FD-based multi-wavelength bio-signal analyzing apparatus according to still another embodiment of the present disclosure.

FIG. 21 illustrates an example where eight light emission units and eight detection units are arranged as crossed with each other on a contact surface of a probe.

FIG. 22 is a diagram showing the relationship between a distance between a light emission unit and a detection unit and a depth in a subject according to an embodiment of the present disclosure.

FIG. 23 illustrates an example where light emission units and detection units are arranged at different distances from each other on a contact surface of a probe.

FIG. 24 is a flowchart provided to explain a method of operating an FD-based multi-wavelength bio-signal analyzing apparatus according to an embodiment of the present disclosure.

FIG. 25 is a flowchart provided to explain a method of operating an FD-based multi-wavelength bio-signal analyzing apparatus according to another embodiment of the present disclosure.

## BEST MODE FOR CARRYING OUT THE INVENTION

A first aspect of the present disclosure provides an FD-based multi-wavelength bio-signal analyzing apparatus including: four or more light sources configured to irradiate frequency-modulated light at four or more different discrete wavelengths; at least one light detector configured to detect an output light reflected and introduced from a subject; and a processing circuit connected to the four or more light sources and the at least one light detector and configured to calculate a scattering coefficient and an absorption coefficient at each discrete wavelength based on an output light and calculate a concentration of a chromophore present in the subject based on the scattering coefficient and the absorption coefficient at each discrete wavelength. Herein, the processing circuit may drive at least two of the four or more light sources based on the chromophore present in the subject.

Further, the processing circuit may determine the number and kinds of light sources to be driven from among the four or more light sources based on at least one of the number, kind, and content of the chromophore present in the subject.

Furthermore, the chromophore present in the subject may include at least one of oxy-hemoglobin (O2Hb), deoxy-hemoglobin (HHb), water (H2O), and lipid.

Moreover, the processing circuit may calculate the scattering coefficient and the absorption coefficient at each discrete wavelength by fitting an amplitude and phase of the output light into a diffusion model, and calculate a concentration value of the chromophore present in the subject by fitting the calculated result into an already-known extinction coefficient spectrum of the chromophore present in the subject.

Further, the processing circuit may perform calibration to compensate for a phase and amplitude of a signal caused by the device itself from the output light.

Furthermore, the four or more different discrete wavelengths may be discontinuous wavelengths in a near infrared ray region. Moreover, the four or more different discrete wavelengths may be determined based on an already-known absorbance of a chromophore present in the body.

Further, the four or more different discrete wavelengths may include a first discrete wavelength and a second discrete wavelength adjacent to peak regions in already-known absorption spectra of water (H2O) and lipid, respectively, and a third discrete wavelength before an isosbestic point and a fourth discrete wavelength of a region adjacent to the isosbestic point in already-known absorption spectra of oxy-hemoglobin (O2Hb) and deoxy-hemoglobin (HHb).

Furthermore, the four or more different discrete wavelengths may include wavelengths of about 688 nm, about 808 nm, about 915 nm, and about 975 nm.

Moreover, the light source may be implemented as a laser diode (LD) or light emitting diode (LED), and four or more LDs may be implemented as multi-wavelengths vertical cavity surface emitting laser devices (VCSELs), respectively. Otherwise, each of the LDs may be implemented as a vertical cavity surface emitting laser device (VCSEL), and the four or more vertical cavity surface emitting laser devices (VCSELs) may be arranged spaced from each other on a contact surface of a probe.

Further, the at least one light detector may include at least one avalanche photodiode (APD).

Furthermore, the processing circuit may sequentially drive the two or more light sources.

Moreover, the processing circuit may drive a light source that emits light at the shortest wavelength first from among the two or more light sources and then drive light sources that emit light at a gradually longer wavelength.

Further, the processing circuit may regulate the intensities of light output from the two or more light sources depending on at least one of mechanical properties and surrounding environments of the light sources.

Furthermore, the FD-based diffuse optical spectroscopic imaging apparatus may further include: a housing including the four or more light sources, the at least one light detector, and the processing circuit; a first optical fiber coupled to the four or more light sources and configured to concentrate and transmit light irradiated from the four or more light sources; and a second optical fiber coupled to at least one light detector and configured to concentrate light reflected from the subject and transmit the light to the at least one light detector. Herein, the first optical fiber and the second optical fiber may be exposed to the outside of the housing to be in contact with the subject.

Moreover, the FD-based multi-wavelength bio-signal analyzing apparatus may further include a probe including the four or more light sources, the at least one light detector, and a contact surface in contact with the subject. Herein, the four or more light sources may be arranged on the contact surface of the probe and may directly irradiate two or more lights driven by the processing circuit.

Further, the probe may further include a lens arranged on the contact surface of the probe and combined with the four or more light sources.

Furthermore, the FD-based multi-wavelength bio-signal analyzing apparatus may further include a probe including multiple light emission unit including the four or more light sources, multiple detection units including the at least one light detector, and a contact surface in contact with the subject. Herein, the multiple light emission units and the multiple detection units may be arranged as crossed with each other on the contact surface of the probe.

Moreover, the processing circuit may sequentially drive the multiple light emission units, and if one of the multiple light emission units is driven, the processing circuit may drive at least one detection unit adjacent to the driven light emission unit to detect an output light.

Further, the processing circuit may sequentially drive the multiple light emission units, and if one of the multiple light emission units is driven, the processing circuit may drive two or more detection units arranged at different distances from the driven light emission unit to detect an output light.

Furthermore, the multiple light emission units and the multiple detection units may be arranged at different distances as crossed with each other on the contact surface of the probe.

Moreover, the FD-based multi-wavelength bio-signal analyzing apparatus may include four light sources, five light sources, six light sources, seven light sources, or eight light sources.

A second aspect of the present disclosure provides an FD-based multi-wavelength bio-signal analyzing apparatus including: four or more light sources configured to emit light at four or more different discrete wavelengths; at least one light detector configured to detect an output light reflected and introduced from a subject; a processing circuit connected to the four or more light sources and the at least one light detector and configured to calculate a scattering coefficient and an absorption coefficient at each discrete wavelength based on an output light detected by the at least one light detector and calculate a concentration of a chromophore present in the subject based on the scattering coefficient and the absorption coefficient at each discrete wavelength; a first optical fiber coupled to the four or more light sources and configured to concentrate and transmit light irradiated from the four or more light sources; a second optical fiber coupled to at least one light detector and configured to concentrate light reflected from the subject and transmit the light to the at least one light detector; and a housing including the four or more light sources, the at least one light detector, and the processing circuit. Herein, the first optical fiber and the second optical fiber may be exposed to the outside of the housing to be in contact with the subject.

Further, a third aspect of the present disclosure provides an FD-based multi-wavelength bio-signal analyzing apparatus including: at least one light emission unit in which four or more light sources configured to emit light at four or more different discrete wavelengths are arranged spaced at a predetermined distance from each other; at least one detection unit including at least one light detector configured to detect an output light emitted from a subject; a probe including the at least one light emission unit and the at least one detection unit and having a contact surface in contact with the subject; and a processing circuit connected to the at least one light emission unit and the at least one detection unit and configured to calculate a scattering coefficient and an absorption coefficient at each discrete wavelength based on an output light detected by the at least one detection unit and calculate a concentration of a chromophore present in the subject based on the scattering coefficient and the absorption coefficient at each discrete wavelength. Herein, the at least one light emission unit and the at least one detection unit may be arranged on the contact surface of the probe to be in direct contact with the subject.

A fourth aspect of the present disclosure provides a method of operating an FD-based multi-wavelength bio-signal analyzing apparatus, including: driving at least two of four light sources configured to irradiate frequency-modulated light at four or more different discrete wavelengths based on a chromophore present in a subject; detecting two or more output lights reflected and introduced from the subject when the two or more light sources are driven; calculating a scattering coefficient and an absorption coefficient at each discrete wavelength based on the two or more output lights; and calculating a concentration of the chromophore present in the subject based on the scattering coefficient and the absorption coefficient at each discrete wavelength.

A fifth aspect of the present disclosure provides an FD-based multi-wavelength bio-signal analyzing apparatus including: four or more LDs configured to irradiate frequency-modulated light at four or more different discrete wavelengths determined based on a chromophore present in a subject; at least one light detector configured to detect an output light emitted from the subject; and a processing circuit connected to the four or more LDs and the at least one light detector and configured to sequentially drive the four or more LDs, obtain four or more output lights detected by the at least one light detector when the four or more LDs are sequentially driven, calculate a scattering coefficient and an absorption coefficient at each discrete wavelength based on the four or more output lights, and calculate a concentration of the chromophore present in the subject based on the scattering coefficient and the absorption coefficient at each discrete wavelength.

Further, a sixth aspect of the present disclosure provides a method of operating an FD-based multi-wavelength bio-signal analyzing apparatus, including: sequentially driving four or more LDs configured to emit light at four or more different discrete wavelengths determined based on a chromophore present in a subject; sequentially obtaining four or more output lights emitted from the subject when the four or more LDs are sequentially driven; calculating a scattering coefficient and an absorption coefficient at each discrete wavelength based on the four or more output lights; and calculating a concentration of the chromophore present in the subject based on the scattering coefficient and the absorption coefficient at each discrete wavelength.

Furthermore, a seventh aspect of the present disclosure provides a computer-readable storage medium in which a program configured to implement the fourth to sixth aspects is stored.

## MODE FOR CARRYING OUT THE INVENTION

Hereinafter, embodiments of the present disclosure will be described in detail with reference to the accompanying drawings so that the present disclosure may be readily implemented by a person with ordinary skill in the art. However, it is to be noted that the present disclosure is not limited to the embodiments but can be embodied in various other ways. In drawings, parts irrelevant to the description are omitted for the simplicity of explanation, and like reference numerals denote like parts through the whole document.

Further, in the following explanation with reference to the accompanying drawings, components bearing the same name may be assigned different reference numerals depending on a drawing. A reference numeral is assigned only for convenience in explanation, and a concept, feature, function, or effect of each component should not be limitedly construed by the corresponding reference numeral.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Furthermore, through the whole document, the term "object" refers to a target to be measured by a frequency domain (FD)-based multi-wavelength bio-signal analyzing apparatus of the present disclosure and may include a person or animal or a part thereof. Also, the object may include various organs such as the heart, brain, or blood vessels or a variety of phantoms.

Hereinafter, an embodiment of the present disclosure will be described in detail with reference to the accompanying drawings.

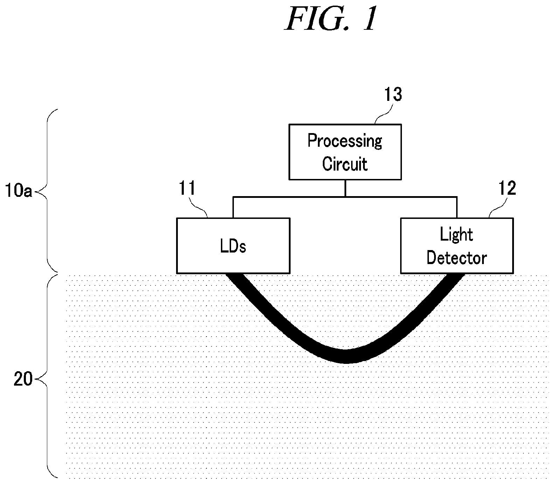

FIG. 1 is a diagram illustrating a configuration of a frequency domain-based multi-wavelength bio-signal analyzing apparatus (hereinafter, referred to as "bio-signal analyzing apparatus") according to an embodiment of the present disclosure. A bio-signal analyzing apparatus 10 according to an embodiment of the present disclosure is implemented using a limited number of light sources. Herein, the light sources may be implemented as laser diodes (LDs) or light emitting diodes (LEDs) capable of irradiating frequency-modulated light. In the following description, an example where the light sources are implemented as LDs will be described.

As illustrated in FIG. 1, the bio-signal analyzing apparatus 10 according to an embodiment of the present disclosure includes four or more LDs 11, at least one light detector 12, and a processing circuit 13.

The four or more LDs 11 irradiate frequency-modulated light at different discrete wavelengths. Herein, the discrete wavelengths may refer to discontinuous wavelengths in a near infrared ray region. For example, the four or more LDs 11 may emit light at a wavelength ranging from 650 nm to 1,100 nm (nano-meter).

Further, the different discrete wavelengths are determined based on a chromophore present in a subject 20. To be specific, the different discrete wavelengths may be determined based on an already-known absorption of each chromophore. Herein, the chromophore refers to an atom or group of atoms that absorbs light. In general, the kinds of chromophores present in the body are limited and already known. For example, water ($H_2O$), lipid, oxy-hemoglobin ($O_2Hb$), and deoxy-hemoglobin (HHb) are dominantly present in the tissues of arms, legs, etc., and $H_2O$, oxy-hemoglobin, and deoxy-hemoglobin except lipid are dominantly present in the brain.

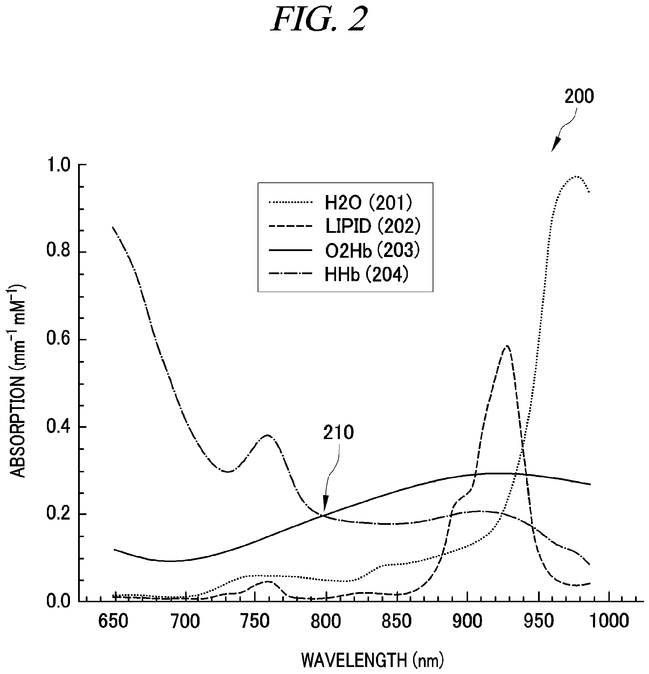

FIG. 2 is a graph 200 showing absorption spectra of chromophores present in the body. In general, chromophores have their own absorption spectrum in a near infrared ray region. To be specific, as shown in FIG. 2, water 201 shows a peak around the 980 nm wavelength region and lipid 202 shows a peak around the 930 nm wavelength region. Further, oxy-hemoglobin 203 and deoxy-hemoglobin 204 intersect each other at an isosbestic point 210 around the 800 nm wavelength region.

According to an embodiment, the bio-signal analyzing apparatus 10 may be implemented using four LDs and may irradiate frequency-modulated light at four discrete wavelengths determined based on the absorbance of water, lipid, oxy-hemoglobin, and deoxy-hemoglobin. To be specific, the four discrete wavelengths include a first discrete wavelength adjacent to the peak region of the water 201 and a second discrete wavelength adjacent to the peak region of the lipid 202, and may include a third discrete wavelength before the isosbestic point 210 of the already-known absorption spectrum of the oxy-hemoglobin 203 and the deoxy-hemoglobin 204 and a fourth discrete wavelength in a region adjacent to the isosbestic point 210. Herein, the third discrete wavelength may be selected from a region in which the oxy-hemoglobin 203 and the deoxy-hemoglobin 204 have a relatively big difference in absorption in consideration of the absorbance of the deoxy-hemoglobin 204. For example, the first discrete wavelength may be about 975 nm and the second discrete wavelength may be about 915 nm. Further, the third discrete wavelength and the fourth discrete wavelength may be about 688 nm and about 808 nm, respectively, but may not be limited thereto.

According to another embodiment, the bio-signal analyzing apparatus 10 may be implemented using five, six, seven or eight LDs configured to irradiate light at different wavelengths from the first to fourth discrete wavelengths. Therefore, fifth to eight discrete wavelengths to be added may be determined based on unique properties (e.g., peak) shown in absorption spectra of chromophores other than the above-described chromophores (i.e., water, lipid, oxy/deoxy-hemoglobin). For example, the fifth to eight discrete wavelengths to be added may be determined based on peaks in absorption spectra of collagen, melanin, methemoglobin (MetHb), or CO hemoglobin (COHb) other than the above-described chromophores. However, the present disclosure may not be limited thereto, and wavelengths to be added can be selected in consideration of various conditions. For example, wavelengths to be added may be selected based on the centers of gravity of absorption spectra of chromophores.

As such, the bio-signal analyzing apparatus 10 includes four or more LDs determined based on unique properties shown in absorption spectra of chromophores present in the body, and, thus, the processing circuit 13 can calculate a concentration of each chromophore with more accuracy.

Meanwhile, the bio-signal analyzing apparatus 10 has been described above as implemented using four LDs, five LDs, six LDs, seven LDs, or eight LDs, but may not be limited thereto. In some embodiments, the bio-signal analyzing apparatus 10 may be implemented using a smaller or greater number of LDs.

The light detector 12 is configured to detect an output light reflected and introduced from the subject 20 under the control of the processing circuit 13. The light detector 12 may convert the detected output light into an electrical signal and provide the electrical signal to the processing circuit 13.

The light detector 12 may be implemented using at least one avalanche photodiode (APD), but may not be limited thereto. The light detector 12 may be implemented into various forms, such as a photodiode, a photo transistor, a photo multiplier tube (PMT), a photo cell), and the like. Further, the light detector 12 may be implemented including a new type of photo sensor developed with advances of technology.

Further, the light detector 12 may be arranged at a predetermined distance from the four or more LDs 11 to measure light emitted and introduced from the subject.

Meanwhile, the four or more LDs 11 and the at least one light detector 12 may be implemented by heterodyne technique in which frequency-modulated light is irradiated and detected using an intermediate frequency (IF), or may be implemented by homodyne technique in which a modulated frequency of light is directly converted into a baseband one and detected.

The processing circuit 13 is configured to control overall operations of the bio-signal analyzing apparatus 10. For example, the processing circuit 13 may execute a bio-signal analyzing program stored in a memory (not illustrated) to control the four or more LDs 11 and the at least one light detector 12. In this case, the processing circuit 13 may be implemented as a processor used in a general purpose computing device or as an embedded processor.

The processing circuit 13 may execute the program to control driving of the four or more LDs 11, calculate a scattering coefficient and an absorption coefficient at each discrete wavelength based on an output light detected by the at least one light detector 12, and measure a concentration of a chromophore present in the subject 20 to analyze the biological composition of the subject 20.

Firstly, the processing circuit 13 may drive two or more of the four or more LDs 11 based on at least one of the number, content, and kind of at least one chromophore present in the subject 20. That is, the processing circuit 13 may determine the number and kinds of LDs to be driven from among the four or more LDs 11.

For example, if the number of chromophores present in the subject 20 is four, the processing circuit 13 may determine four LDs based on unique properties of the chromophores shown in the respective absorption spectra. However, even though the number of chromophores present in the subject 20 is four, if it is already known that a specific chromophore is scarcely present (or present in a small content), the processing circuit 13 may determine three LDs based on unique properties of the other three chromophores. For more specific example, in the case where the head of a human is measured, the processing circuit 13 may select three LDs that emit light at first, third, and fourth discrete wavelengths except an LD configured to emit light at a second discrete wavelength and determined based on a peak of lipid from among the four or more LDs 11.

The processing circuit 13 may regulate the intensities of light output depending on mechanical properties and surrounding environments of the respective LDs. For example, the processing circuit 13 may regulate the intensities of light output in consideration of the periods of use of the respective LDs, the amounts of current supplied to the respective LDs, and the ambient light.

The processing circuit 13 may sequentially drive the selected two or more LDs. For example, the processing circuit 13 may drive an LD that emits light at the shortest wavelength first from among the selected two or more LDs and then drive LDs that emit light at a gradually longer wavelength, but may not be limited thereto. For example, the processing circuit 13 may sequentially drive the selected two or more LDs in the sequence of arrangement.

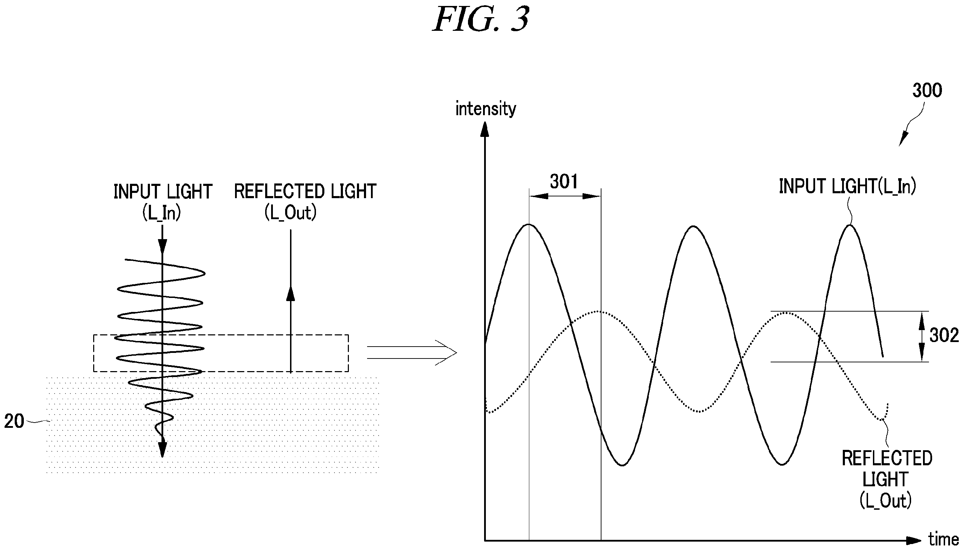

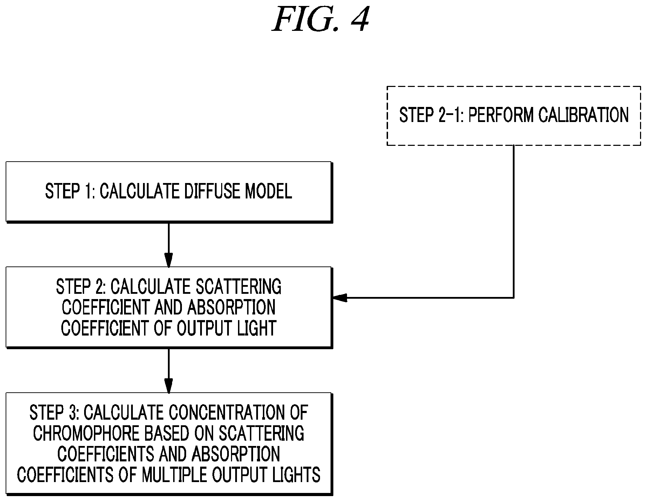

Then, the processing circuit 13 may drive the light detector 12 to receive an output light detected by the light detector 12. Then, the processing circuit 13 may calculate an absorption coefficient and a scattering coefficient at each discrete wavelength based on the output light. Details thereof will be described with reference to **FIG. 3** and **FIG. 4.**

**FIG. 3** shows characteristic of an input light input into the subject 20 by the LD and an output light detected by a light detector 12 according to an embodiment of the present disclosure.

As shown in a right diagram in **FIG. 3,** if a frequency-modulated input light is irradiated from an LD into the subject 20, the input light is scattered and absorbed by various components including the chromophore present in the subject 20.

A graph 300 shown on the left of **FIG. 3** shows properties of an input light L_In and an output light (i.e., reflected light) L_Out in the frequency domain FD. As the frequency-modulated input light L_In is irradiated from the LD into the subject 20, the reflected light L_Out detected by the light detector 12 shows a phase shift 301 and an amplitude attenuation 302 with respect to the input light L_In.

The processing circuit 13 calculates an absorption coefficient and a scattering coefficient at each discrete wavelength using the phase shift 301 and the amplitude attenuation 302 occurring at each discrete wavelength, and calculates a concentration value of each chromophore based on the calculated absorption coefficient and scattering coefficient. To this end, the processing circuit 13 may use diffuse approximation of the radiative transfer equation.

FIG. 4 is a diagram showing a process of calculating a concentration of each chromophore by the processing circuit 13 illustrated in **FIG. 1.**

STEP 1: The processing circuit 13 obtains a diffusion model of a frequency domain calculated using the Green's function in the diffusion approximation. Herein, the diffusion model uses an extrapolated boundary condition as a sample (subject)-air boundary condition. Therefore, the energy fluence at a certain distance $Z_b$ from a surface of the sample is assumed to be 0. $Z_b$ can be defined as shown in the following Equation 1.

[Equation 1]

$$Z_b = 2D(1+R_{eff})/(1-R_{eff})$$

In the above Equation 1, $R_{eff}$ represents an effective reflectance which is affected by a refractive index. If the sample has a refractive index of 1.4 and the air has a refractive index of 1.0, $R_{eff}$ may be 0.493. Further, $D$ represents a diffusion coefficient and is defined as $l_{tr}/3$. Herein, $l_{tr}$ can be defined as shown in the following Equation 2.

[Equation 2]

$$l_{tr}\,(transport\ mean\ free\ path) = (\mu_a + \mu_s')^{-1}$$

Meanwhile, the diffusion model may be previously stored in the memory (not illustrated) of the bio-signal analyzing apparatus 10.

STEP 2: Then, the processing circuit 13 measures an optical signal based on the frequency domain. The processing circuit 13 measures an output light corresponding to Equation 3 based on the frequency domain.

[Equation 3]

$$R\,(Reflectance\ Signal) = C_0 \cdot A \cdot exp(-i(\varphi + \varphi_0)$$

In the above Equation 3, $R$ represents the measured output light and $A$ and $\varphi$ represent amplitude and phase components of a signal reflected and introduced from the subject in the measured output light. Further, $C_0$ and $\varphi_0$ represent the amplitude and phase included in the output light due to device itself regardless of a subject. $C_0$ and $\varphi_0$ are calculated by calibration in the following STEP 2-1.

STEP 2-1: The processing circuit 13 can calculate values of $C_0$ and $\varphi_0$ before measuring a subject. To be specific, the processing circuit 13 may measure a subject with the already-known absorption coefficient $\mu_a$ and scattering coefficient $\mu_s'$ and predict the amplitude and phase of an output light reflected from the subject. Then, the processing circuit 13 substitutes the amplitudes and phases of the measured output light and the predicted output light in Equation 3 to obtain $C_0$ and $\varphi_0$. However, in some embodiments, the processing circuit 13 may do not perform the operation of STEP 2-1. In this case, the processing circuit 13 may receive the already determined $C_0$ and $\varphi_0$.

Referring to STEP-2 again, the processing circuit 13 compensates for error values caused by the device itself (i.e., phase and amplitude caused by the device) from the measured output light $R$ using the previously obtained $C_0$ and $\varphi_0$. Then, the processing circuit 13 can calculate an absorption coefficient $\mu_a$ and a scattering coefficient $\mu_s'$ based on the amplitude A and the phase $\varphi$ of the output light R obtained according to Equation 3. To be specific, the processing circuit 13 can calculate the absorption coefficient $\mu_a$ and the scattering coefficient $\mu_s'$ of a measurement subject corresponding to each wavelength of the output light by fitting the amplitude and phase (STEP 1) of the output light into a diffusion model. Herein, the processing circuit 13 may perform least square fitting to the amplitude and phase of the output light.

The processing circuit 13 can calculate an absorption coefficient $\mu_a$ and a scattering coefficient $\mu_s'$ at each of different discrete wavelengths by repeatedly performing the above-described process to output lights to be detected by the sequentially driven LDs.

STEP 3: The processing circuit 13 can calculate concentration values of chromophores based on already-known extinction coefficient spectra of the chromophores using the calculated absorption coefficient and scattering coefficient at each discrete wavelength.

The processing circuit 13 can analyze constituent components in the subject 20 using the concentrations of the respective chromophore.

As described above, the bio-signal analyzing apparatus 10 according to an embodiment of the present disclosure provides a method of measuring a concentration of a chromophore using a limited number of LDs.

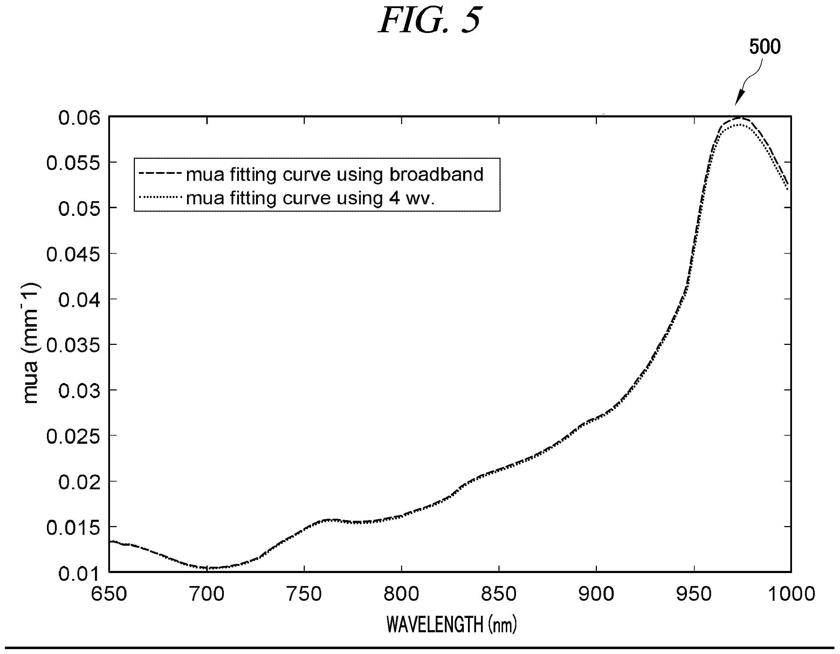

**FIG. 5** to **FIG. 8** are graphs showing the result of comparison between an absorption coefficient spectrum obtained according to an embodiment of the present disclosure and an absorption coefficient spectrum which is reconstructed with a chromophore concentration obtained by fitting from an absorption coefficient obtained by an FD-SS (steady state) diffuse optical spectroscopic imaging apparatus (see U.S. Patent No. 7,428,434). Meanwhile, **FIG. 5** to **FIG. 8** shows the result of measuring a woman's breast tissue.

To be specific, **FIG. 5** is a graph 500 comparing an absorption coefficient spectrum extracted by the process illustrated in **FIG. 4** from an output light detected by four LDs that irradiate frequency-modulated light at discrete wavelengths of about 688 nm, about 808 nm, about 915 nm, and about 975 nm with an absorption coefficient spectrum calculated by the FD-SS diffuse optical spectroscopic imaging apparatus.

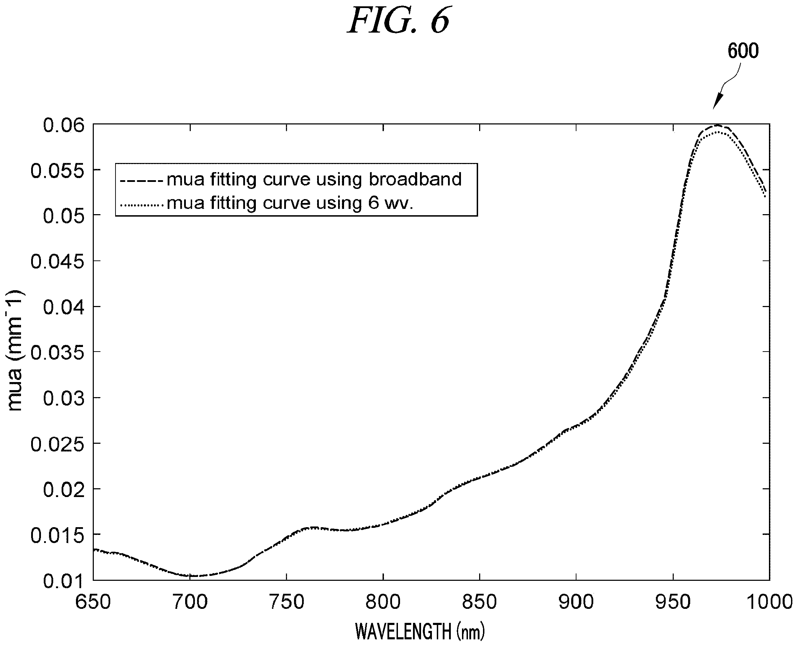

**FIG. 6** is a graph 600 comparing an absorption coefficient spectrum extracted by the process illustrated in **FIG. 4** from an output light detected by five LDs that irradiate frequency-modulated light at discrete wavelengths of about 688 nm, about 808 nm, about 860 nm, about 915 nm, and about 975 nm with an absorption coefficient spectrum calculated by the FD-SS diffuse optical spectroscopic imaging apparatus.

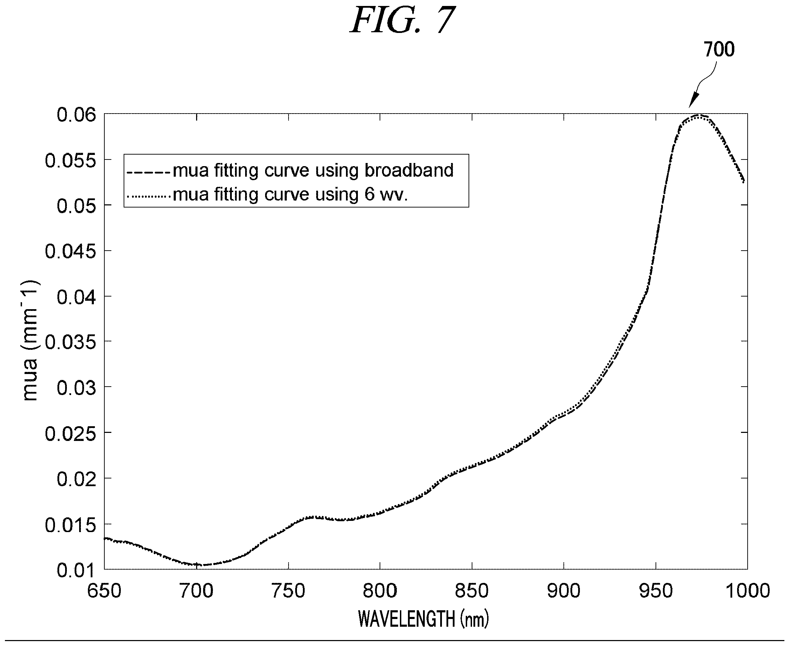

Further, **FIG. 7** is a graph 700 comparing an absorption coefficient spectrum extracted by the process illustrated in **FIG. 4** from an output light detected by six LDs that irradiate frequency-modulated light at discrete wavelengths of about 688 nm, about 705 nm, about 808 nm, about 860 nm, about 915 nm, and about 975 nm with an absorption coefficient spectrum calculated by the FD-SS diffuse optical spectroscopic imaging apparatus.

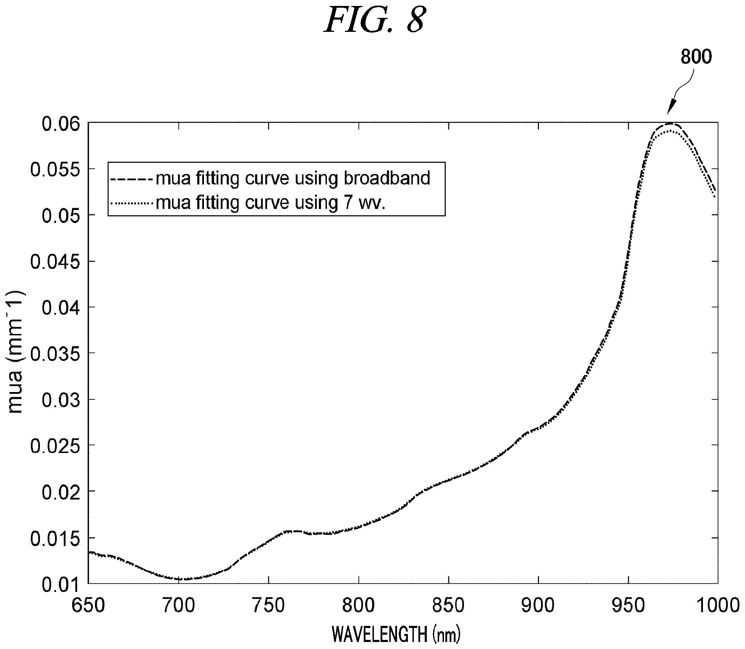

Furthermore, **FIG. 8** is a graph 800 comparing an absorption coefficient spectrum extracted by the process illustrated in **FIG. 4** from an output light detected by seven LDs that irradiate frequency-modulated light at discrete wavelengths of about 688 nm, about 705 nm, about 785 nm, about 808 nm, about 860 nm, about 915 nm, and about 975 nm with an absorption coefficient spectrum calculated by the FD-SS diffuse optical spectroscopic imaging apparatus.

Meanwhile, the discrete wavelengths used in **FIG. 5** are determined based on peaks of water, lipid, and oxy/deoxy-hemoglobin, and the discrete wavelengths added in **FIG. 6** to **FIG. 8** are selected randomly to analyze the effect of addition of a wavelength on a result value.

Referring to the graphs 500, 600, 700, and 800 of **FIG. 5** to **FIG. 8**, it can be seen that the absorption coefficient spectrum calculated by the bio-signal analyzing apparatus 10 and the absorption coefficient spectrum calculated by the FD-SS diffuse optical spectroscopic imaging apparatus show almost similar results. The bio-signal analyzing apparatus 10 of the present disclosure is advantageous in that the FD-SS diffuse optical spectroscopic imaging apparatus detects and analyzes data in broadband wavelengths (from about 650 nm to about 1000 nm), whereas the bio-signal analyzing apparatus 10 detects and analyzes data only in a limited number of wavelengths. Further, referring to the graphs 500, 600, 700, and 800 of **FIG. 5** to **FIG. 8,** it can be seen that the absorption coefficient spectrum calculated from the four discrete wavelengths and the absorption coefficient spectra each obtained by adding a certain wavelength show similar results.

The followings are the results of analyzing residuals of the absorption coefficient spectra shown in **FIG. 5** to **FIG. 8** to more accurately show the performance of the bio-signal analyzing apparatus 10. To this end, the analysis results were compared by applying Equation 4 to data constituting the respective spectra.

[Equation 4]

$$R^2 = 1 - SS_{res}/SS_{tot}$$

$$SS_{res} = \sum_i (y_i - f_i)^2 = \sum_i e_i^2$$

$$SS_{\text{tot}} = \sum_i \left( y_i - \bar{y} \right)^2$$

$$\bar{y} = \frac{1}{n} \sum_{i=1}^{n} y_i$$

In the above Equation 4, $y_i$ represents data corresponding to each wavelength for an absorption coefficient spectrum, $\bar{y} = \dfrac{1}{n} \sum_{i=1}^{n} y_i$ represents a mean value of the data, and $f_i$ represents an absorption coefficient fitting value reconstructed based on a concentration value of a chromophore. Further, $SS_{\text{tot}} = \sum_i \left( y_i - \bar{y} \right)^2$ repre-sents the sum of squares of values obtained by subtracting the mean value of the data from the data, and

$$SS_{\text{res}} = \sum_i \left( y_i - f_i \right)^2 = \sum_i e_i^2$$ represents the sum of squares of values obtained by subtracting the fitting value from the data.

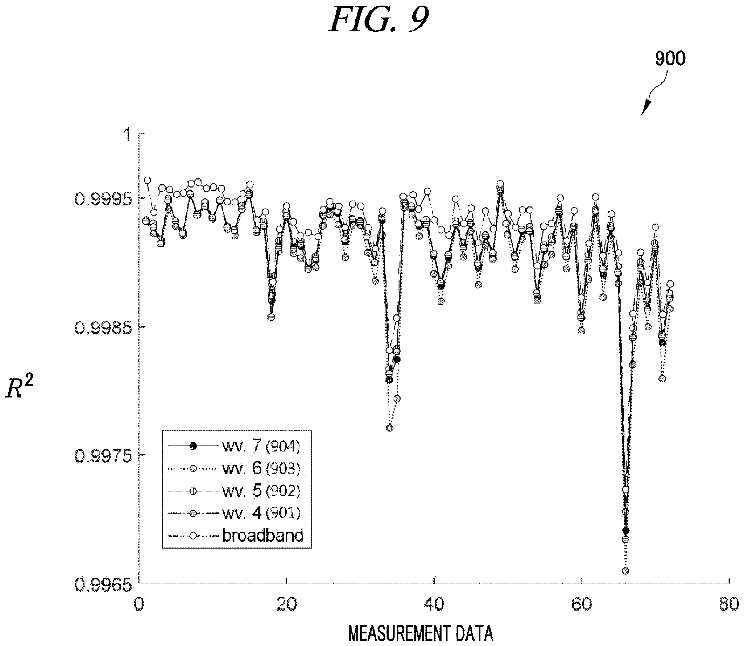

**FIG. 9** is a graph showing the result of $R^2$ analysis on a total of 72 data of the absorption coefficient spectra used in **FIG. 5** to **FIG. 8.**

In **FIG. 9,** dots corresponding to the "broadband" represent the result values obtained by the FD-SS diffuse optical spectroscopic imaging apparatus. The FD-SS diffuse optical spectroscopic imaging apparatus calculates an absorption coefficient spectrum using a broadband wavelength and thus has the greatest $R^2$ value. However, it can be seen that all of the results obtained by the bio-signal analyzing apparatus 10 according to an embodiment of the present disclosure are also included in the range of from 1 to 0.990. Therefore, the dots confirm that the results obtained by the FD-SS diffuse optical spectroscopic imaging apparatus are similar to those obtained by the bio-signal analyzing apparatus 10.

Further, it can be seen that an $R^2$ value 901 obtained when four discrete wavelengths are driven is not much different from $R^2$ values 902, 903, and 904 obtained when four or more discrete wavelengths are driven. The measurement results shown in **FIG. 5** to **FIG. 9** confirm that the number of discrete wavelengths does not make a big difference in calculating a concentration of each chromophore.

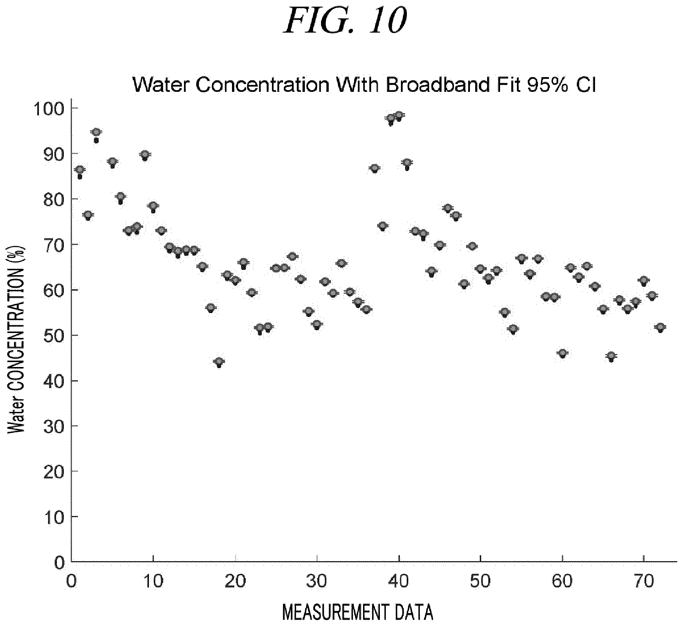

**FIG. 10** to **FIG. 13** are graphs showing concentration values of the respective chromophores calculated from the absorption coefficient spectra used in **FIG. 5** to **FIG. 8.**

**FIG. 10** is a graph showing a concentration value (%) of water ($H_2O$). In **FIG. 10,** circular figures represent concentration values of $H_2O$ calculated by the FD-SS diffuse optical spectroscopic imaging apparatus and a 95% confidence interval for the concentration values. Further, more deeply-colored dots illustrated around the figures represent concentration values of $H_2O$ calculated by the bio-signal analyzing apparatus 10 according to an embodiment of the present disclosure.

Referring to the graph shown in **FIG. 10,** most of the concentration values of $H_2O$ calculated by the bio-signal analyzing apparatus 10 are included in a 95% confidence interval.

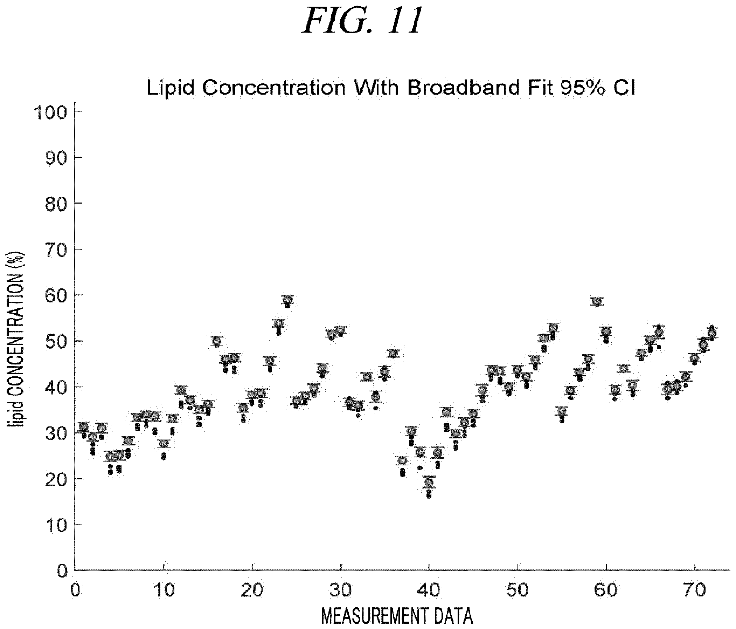

**FIG. 11** is a graph showing a concentration value (%) of lipid. In **FIG. 11,** circular figures represent concentration values of lipid calculated by the FD-SS diffuse optical spectroscopic imaging apparatus and a 95% confidence interval for the concentration values. Further, more deeply-colored dots illustrated around the figures represent concentration values of lipid calculated by the bio-signal analyzing apparatus 10 according to an embodiment of the present disclosure.

Referring to the graph shown in **FIG. 11,** most of the concentration values of lipid calculated by the bio-signal analyzing apparatus 10 are not illustrated as included in a 95% confidence interval. However, the actually measured concentration values had a difference ranging of from about - 4% to about +4%, which did not actually show a big difference.

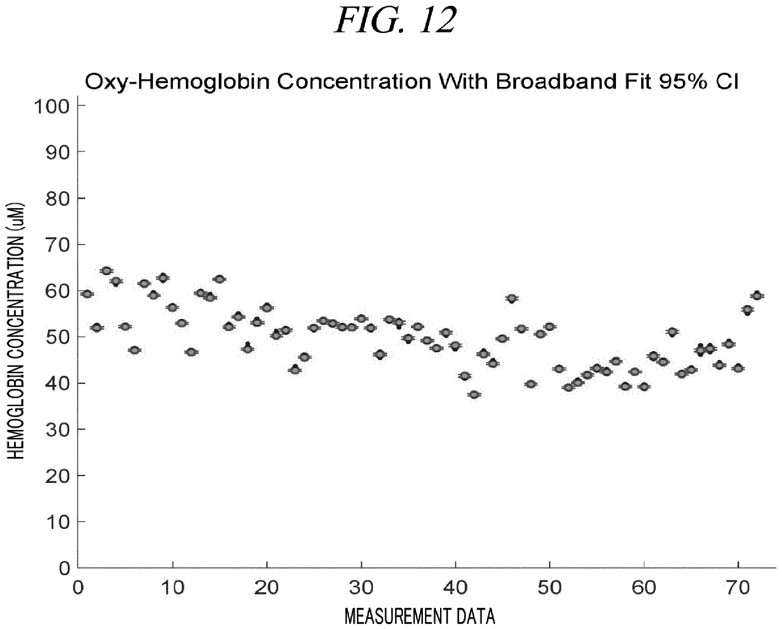

**FIG. 12** is a graph showing a concentration value (uM) of oxy-hemoglobin (O2Hb). In **FIG. 12,** circular figures represent concentration values of oxy-hemoglobin calculated by the FD-SS diffuse optical spectroscopic imaging apparatus and a 95% confidence interval for the concentration values. Further, more deeply-colored dots illustrated around the figures represent concentration values of oxy-hemoglobin calculated by the bio-signal analyzing apparatus 10 according to an embodiment of the present disclosure.

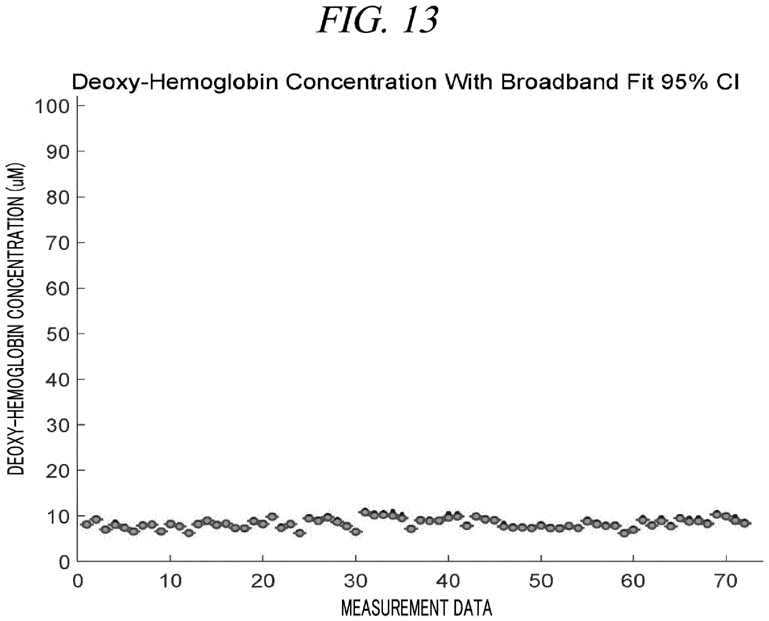

**FIG. 13** is a graph showing a concentration value (uM) of deoxy-hemoglobin (HHb). In **FIG. 13,** circular figures represent concentration values of deoxy-hemoglobin calculated by the FD-SS diffuse optical spectroscopic imaging apparatus and a 95% confidence interval for the concentration values. Further, more deeply-colored dots illustrated around the figures represent concentration values of deoxy-hemoglobin calculated by the bio-signal analyzing apparatus 10 according to an embodiment of the present disclosure. If the concentration values of deoxy-hemoglobin calculated by the bio-signal analyzing apparatus 10 are in the same range as the concentration values of deoxy-hemoglobin calculated by the FD-SS diffuse optical spectroscopic imaging apparatus, the dots are shown as overlapping with the figures.

As shown in **FIG. 12** and **FIG. 13,** most of the concentration values of oxy/deoxy-hemoglobin calculated by the bio-signal analyzing apparatus 10 are included in a 95% confidence interval.

As described above, the bio-signal analyzing apparatus 10 according to an embodiment of the present disclosure can calculate concentration values of chromophores in the subject 20 with high confidence by using only a limited number of discrete wavelengths without obtaining and analyzing all of data in broadband wavelengths. Further, the bio-signal analyzing apparatus 10 can analyze constituent components in the subject 20 using the calculated concentration values.

The bio-signal analyzing apparatus 10 implemented including four or more light sources corresponding to water, lipid, and oxy/deoxy-hemoglobin has been described above as an example, but the present disclosure may not be limited thereto. The bio-signal analyzing apparatus 10 may be implemented in further consideration of chromophores such as collagen or melanin. Further, the bio-signal analyzing apparatus 10 can automatically determine a location of the subject 20 in the body by user input or location sensing and can determine two or more light sources to be driven corresponding to a chromophore dominantly present in the location. For example, the bio-signal analyzing apparatus 10 may drive four or more light sources that show unique properties of water, lipid, collagen, and melanin in respective absorption spectra depending on a measurement location.

Otherwise, the bio-signal analyzing apparatus 10 according to another embodiment of the present disclosure may be implemented including a fixed number of light sources depending on a target location in the body. For example, the bio-signal analyzing apparatus 10 may be implemented including three light sources corresponding to water and oxy/deoxy-hemoglobin to be suitable for measuring the head, but may not be limited thereto.

Further, the bio-signal analyzing apparatus 10 has been described above as selecting two or more of four or more LDs. However, the process of selecting LDs may be omitted depending on an embodiment. In this case, the bio-signal analyzing apparatus 10 may drive all of the four or more LDs 11 and then calculate an absorption spectrum excluding a scattering coefficient and an absorption coefficient for a discrete wavelength at which significant data are not obtained.

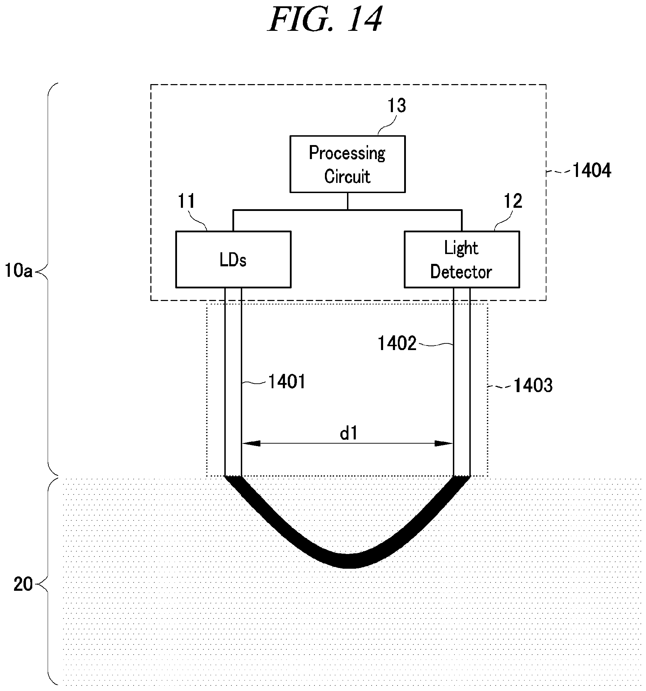

**FIG. 14** is a diagram illustrating a bio-signal analyzing apparatus according to an embodiment of the present disclosure.

Referring to **FIG. 14,** a bio-signal analyzing apparatus 10a includes a housing 1404 including four or more light sources (e.g., LDs 11), at least one light detector 12, and the processing circuit 13 and a probe 1403 exposed to the outside of the housing 1404 to be in contact with a subject and thus to transmit frequency-modulated light irradiated from the LDs 11 to the subject and transmit light reflected from the subject to the light detector 12.

The probe 1403 may include a first optical fiber 1401 coupled to each of the LDs and configured to concentrate and transmit light irradiated from the four or more LDs and a second optical fiber 1402 coupled to the light detector and configured to concentrate an output light and transmit the light to the at least one light detector 12. Herein, a method of coupling the first and second optical fibers 1401 and 1402 to the four or more LDs 11 or the light detector 12 can be easily performed by a person with ordinary skill in the art. Therefore, a detailed explanation thereof will be omitted.

Meanwhile, the first optical fiber 1401 and the second optical fiber 1402 may be arranged spaced at a predetermined distance d1 from each other on a contact surface of the probe 1403 in contact with the subject.

Further, the first optical fiber 1401 may be implemented further including a lens (not illustrated) configured to irradiate light emitted from each LD to the subject 20 at a certain angle.

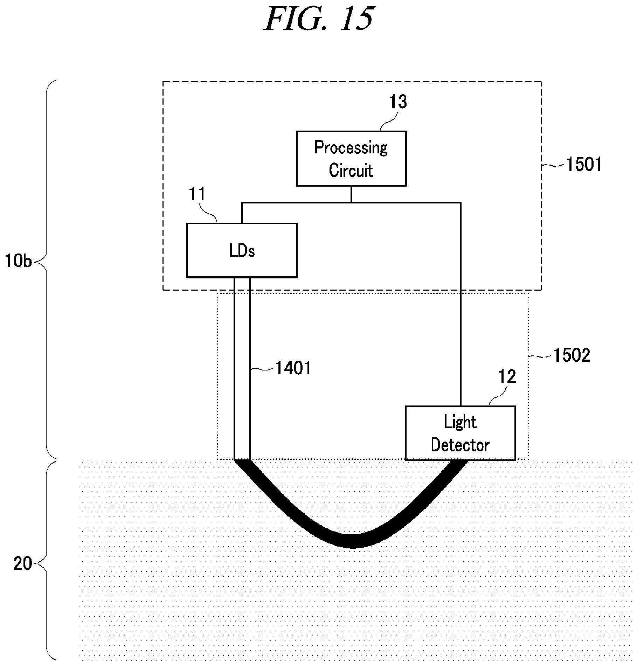

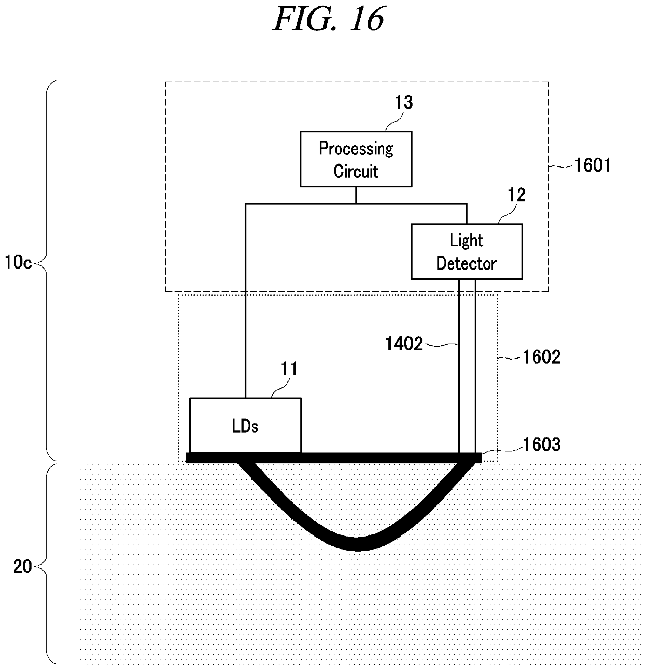

**FIG. 15** and **FIG. 16** are diagrams each illustrating a bio-signal analyzing apparatus according to another embodiment of the present disclosure.

Referring to **FIG. 15,** a bio-signal analyzing apparatus 10b according to an embodiment of the present disclosure may be implemented including a probe 1502 including the first optical fiber 1401 and the light detector 12 illustrated in **FIG. 14** and a housing 1501 connected to the probe 1502 and including four or more light sources (e.g., LDs 11) and the processing circuit 13. The four or more LDs 11 may be configured to irradiate light to a subject through the first optical fiber 1401 and the light detector 12 may be configured to be in direct contact with the subject.

Otherwise, as illustrated in **FIG. 16,** a bio-signal analyzing apparatus 10c according to an embodiment of the present disclosure may be implemented including a probe 1602 including the second optical fiber 1402 and the four or more light sources (e.g., LDs 11) illustrated in **FIG. 14** and a housing 1601 connected to the probe 1602 and including the light detector 12 and the processing circuit 13. The four or more LDs 11 may be configured to be in direct contact with a subject and the light detector 12 may be configured to receive light reflected from the subject through the second optical fiber 1402.

Meanwhile, the four or more LDs may be implemented as multi-wavelength vertical cavity surface emitting laser devices (VCSELs), respectively.

Otherwise, each of the four or more LDs may be implemented as a vertical cavity surface emitting laser device (VCSEL), and the four or more vertical cavity surface emitting laser devices (VCSELs) may be arranged spaced from each other on a contact surface of the probe 1602. In this case, desirably, a space between the four or more LDs 11 can be minimized, and, thus, the processing circuit 13 regards the four or more LDs 11 as emitting light on the same position of the subject 20 and calculate a concentration of a chromophore.

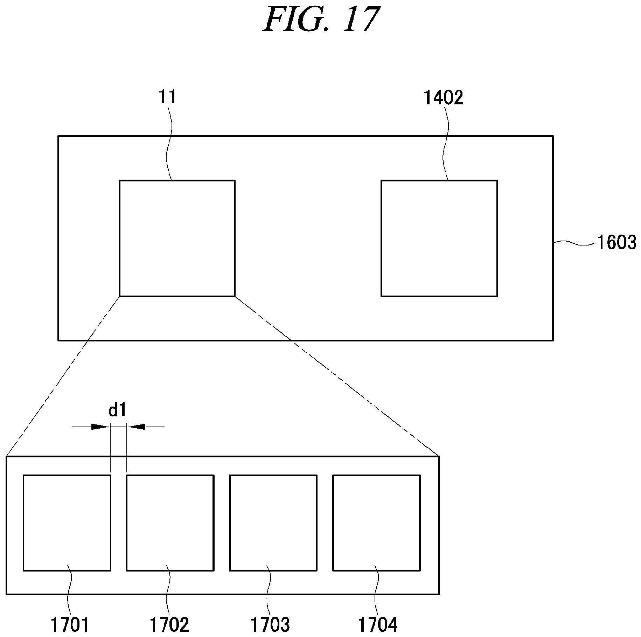

**FIG. 17** is a diagram illustrating a contact surface of the probe illustrated in **FIG. 16.**

Referring to **FIG. 17,** four or more light sources (e.g., LDs 11) in direct contact with the subject 20 and the second optical fiber 1402 may be arranged on a contact surface 1603 of the probe 1602. Light emission elements 1701, 1702, 1703, and 1704 of the respective light sources (i.e., LDs) may be arranged in an integrated manner at a predetermined distance d2 on the contact surface 1603.

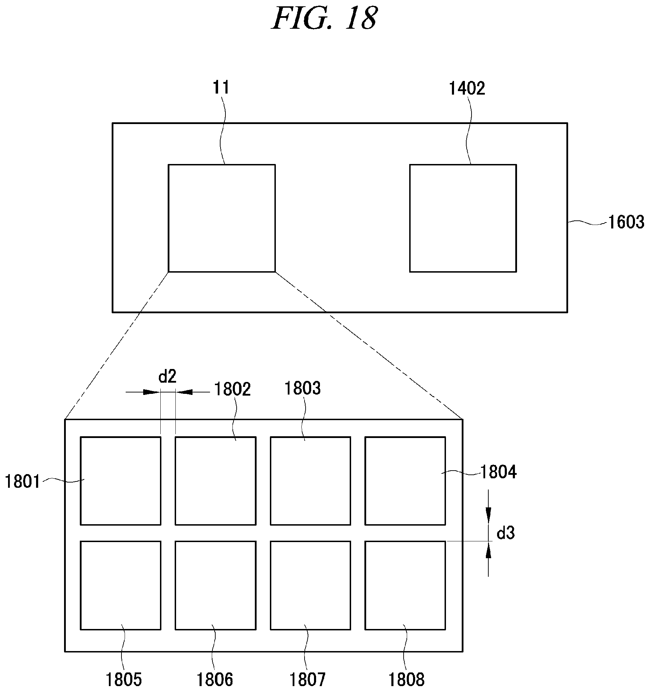

**FIG. 18** is another diagram illustrating a contact surface of the probe illustrated in **FIG. 16.**

Referring to **FIG. 18,** eight light sources (e.g., LDs 11) may be arranged directly on the contact surface 1603 of the probe 1602. In this case, light emission elements 1801, 1802, 1803, 1804, 1805, 1806, 1807, and 1808 of the respective LDs may be arranged in an integrated manner in a 2X4 matrix form, and, thus, a space between the light emission elements of the respective LDs on the contact surface 1603 can be minimized.

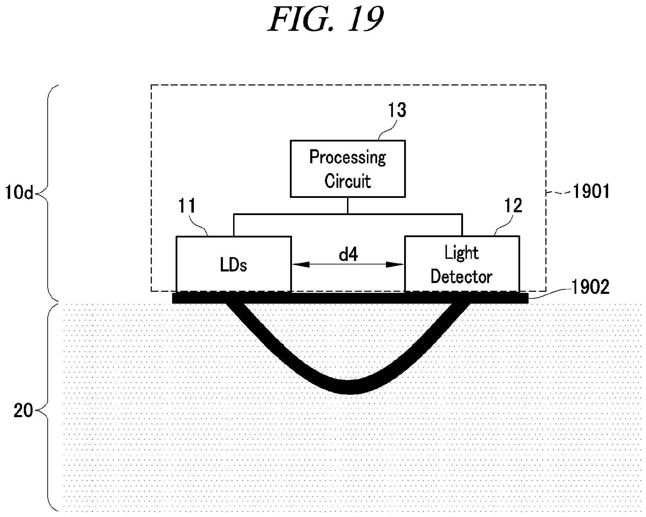

**FIG. 19** is a diagram illustrating a bio-signal analyzing apparatus according to still another embodiment of the present disclosure. Referring to **FIG. 19,** a bio-signal analyzing apparatus 10d may be implemented including a probe 1901 in which four or more light sources (e.g., LDs 11) and the light detector 12 are included. Otherwise, the bio-signal analyzing apparatus 10d may be implemented further including the processing circuit 13 in the probe 1901 depending on an embodiment.

Meanwhile, the four or more LDs 11 and the light detector 12 may be arranged at a predetermined distance d4 on a contact surface 1902 of the probe 1901 and may be in direct contact with the subject 20. Further, the LDs may be arranged spaced at a predetermined distance from each other on the contact surface 1902. The above-described embodiment illustrated in **FIG. 17** and **FIG. 18** can be applied to this embodiment. Therefore, a detailed explanation thereof will be omitted.

Further, the probe 1901 may further include a lens (not illustrated) arranged on the contact surface 1902 and combined with a light source and/or a light detector. The lens may be configured to set light irradiated from the light source toward the same location in the subject 20. Further, the lens can be used to reduce an angle of emission or introduction of light.

As described above, the bio-signal analyzing apparatus 10d can be implemented as the small probe 1901 without an optical fiber and thus can improve the convenience for users in use.

Meanwhile, the bio-signal analyzing apparatus illustrated in **FIG. 1** to **FIG. 19** has been described as performing single channel analysis. However, the bio-signal analyzing apparatus according to an embodiment of the present disclosure may perform multi-channel analysis. Through the multi-channel analysis, the bio-signal analyzing apparatus can analyze the biological composition from various locations at one time without having to shift measurement locations of the bio-signal analyzing apparatus and to scan at those locations. Hereinafter, a bio-signal analyzing apparatus performing multi-channel analysis will be described.

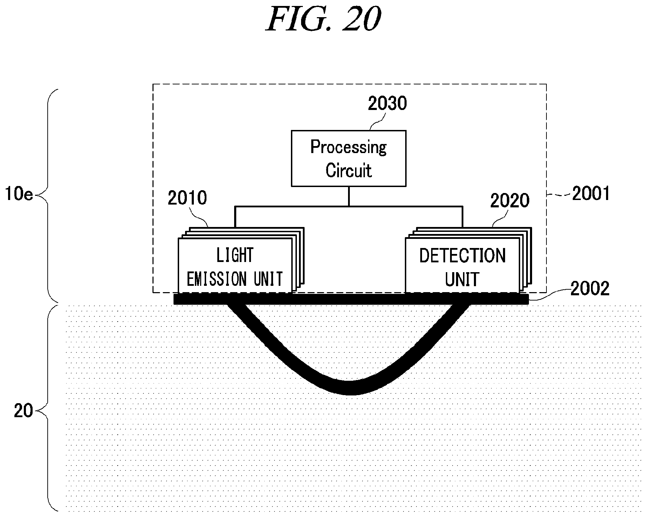

Referring to **FIG. 20,** a bio-signal analyzing apparatus 10e according to an embodiment of the present disclosure includes multiple light emission units 2010 including four or more light sources (e.g., LDs), multiple detection units 2020 including at least one light detector 12, and a processing circuit 2030.

Herein, the multiple light emission units 2010, the multiple detection units 2020, and the processing circuit 2030 may be implemented as a single probe 2001. Further, the multiple light emission units 2010 and the multiple detection units 2020 may be arranged as crossed with each other on a contact surface 2002 of the probe 2001.

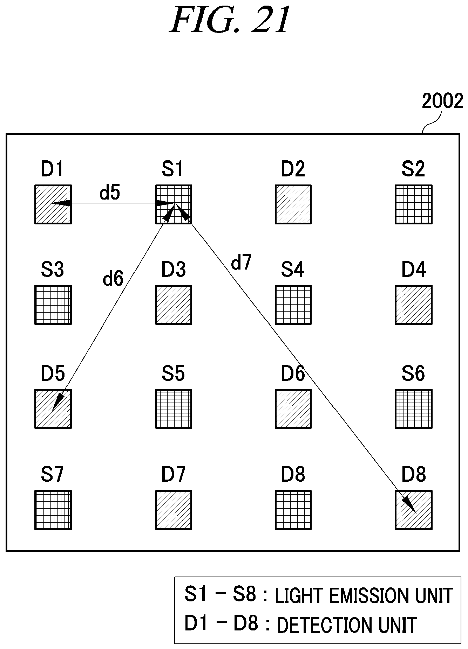

**FIG. 21** illustrates an example where eight light emission units and eight detection units are arranged as crossed with each other on a contact surface 2002 of a probe 20001. As illustrated in **FIG. 21,** in light emission units S1, S2, S3, S4, S5, S6, S7, and S8, four or more LDs may be arranged spaced at a predetermined distance d2 or d3 from each other as illustrated in **FIG. 17** and **FIG. 18.**

Meanwhile, **FIG. 21** illustrates that eight light emission units and eight detection units are arranged in a 4x4 matrix form, but the present disclosure may not be limited thereto. For example, the eight light emission units and the eight detection units may be arranged in a 8X2 matrix form or a 16X1 matrix form.

Referring to **FIG. 20** again, the processing circuit 2030 controls the multiple light emission units 2010 and the multiple detection units 2020. To be specific, the processing circuit 2030 may control the sequence of driving the multiple light emission units 2010 and the multiple detection units 2020. For example, the processing circuit 2030 may sequentially drive the multiple light emission units 2010 and drive at least one detection unit adjacent to each driven light emission unit and thus can analyze biological constituent components corresponding to different locations in the subject 20. For example, referring to **FIG. 21,** the processing circuit 2030 may analyze biological constituent components corresponding to S1 by driving S1 and then driving at least one of D1, D2, and D3 adjacent to S1. Then, the processing circuit 2030 may analyze biological constituent components corresponding to S2 by driving S2 and then driving at least one of D2 and D4 adjacent to S2.

Otherwise, the processing circuit 2030 may sequentially drive the multiple light emission units 2010 and drive two or more detection units arranged at different distances from each driven light emission unit and thus can analyze biological constituent components corresponding to different depths in the subject 20.

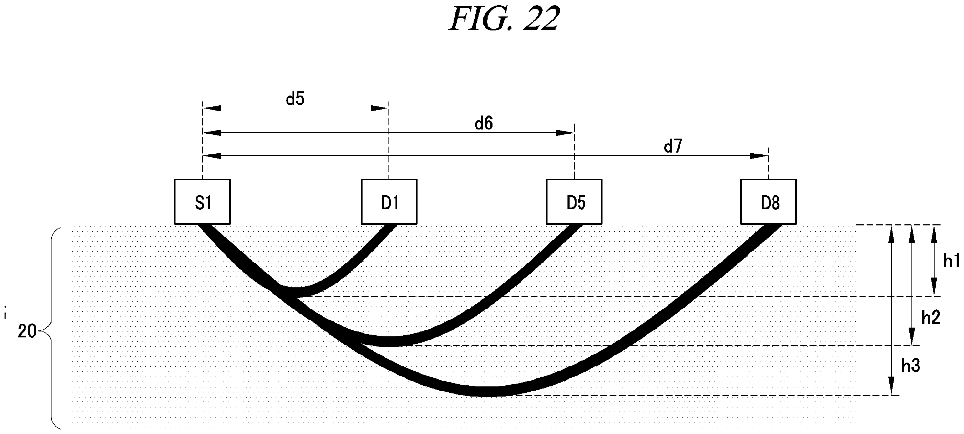

**FIG. 22** is a diagram showing the relationship between a distance between a light emission unit and a detection unit and a measurement depth.

As shown in **FIG. 22,** output lights detected by the respective detection units D1, D5, and D8 correspond to lights absorbed and/or scattered at different depths h1, h2, and h3 in the subject 20 depending on distances d5, d6, and d7 between the light emission unit S1 and the detection units D1, D5, and D8. Therefore, the processing circuit 2030 may analyze biological constituent components corresponding to different depths in the subject 20 by using the output lights detected by the respective detection units D1, D5, and D8 arranged at different distances from the driven light emission unit S1.

Meanwhile, the embodiment illustrated in **FIG. 1** to **FIG. 4** can be applied to a method of controlling each light emission unit and each detection unit and a method of performing bioassay (i.e., measuring a concentration value of a chromophore) based on a detected output light by the processing circuit 2030. Therefore, a detailed explanation thereof will be omitted.

Otherwise, the processing circuit 2030 may analyze biological constituent components corresponding to a specific depth in the subject 20. In this case, the bio-signal analyzing apparatus 10e may receive a user's input to select a specific depth through a user interface (not illustrated). For example, if the user's input to perform bioassay to a depth of less than 0.5 cm in the skin is received, the processing circuit 2030 may drive a detection unit (e.g., D1) adjacent to the light emission unit S1. Otherwise, if the user's input to perform bioassay to a depth of less than 2 cm in the skin is received, the processing circuit 2030 may drive a detection unit (e.g., D5) arranged at a predetermined distance from the light emission unit S1.

Meanwhile, **FIG. 20** to **FIG. 22** illustrate an example where the light emission units and the detection units are arranged at a predetermined distance on the contact surface 2002 of the probe 2001, but the present disclosure may not be limited thereto.

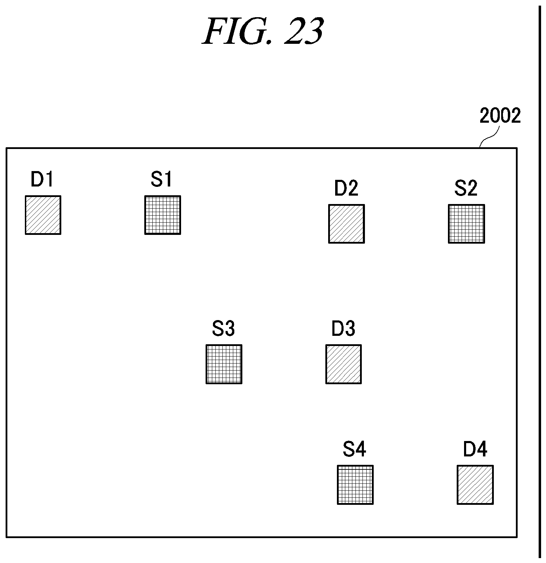

**FIG. 23** illustrates an example where light emission units and detection units are arranged at different distances from each other on the contact surface 2002 of the probe 2001.

Referring to **FIG. 23,** the light emission units and the detection units form pairs D1-S1, D2-S2, D3-S3, and D4-S4 and the respective pairs D1-S1, D2-S2, D3-S3, and D4-S4 may be arranged at different distances from each other. In this case, each of the pairs D1-S1, D2-S2, D3-S3, and D4-S4 may be arranged at the same distance.

The processing circuit 2030 may analyze biological constituent components at multiple measurement locations corresponding to the same depth in the subject 20 by sequentially driving the light emission units and the detection units in the respective pairs D1-S1, D2-S2, D3-S3, and D4-S4.

Further, the processing circuit 2030 may analyze biological constituent components at multiple measurement locations corresponding to different depths, respectively, in the subject 20 by driving a light emission unit each pair and then driving a detection unit in the corresponding pair or another pair.

Meanwhile, the bio-signal analyzing apparatus 10 according to an embodiment of the present disclosure may be implemented including more constituent components in addition to the above-described constituent components. For example, the bio-signal analyzing apparatus 10 may be implemented further including a display (not illustrated) configured to display result values of analysis of biological constituent components (e.g., a concentration of a chromophore) an input unit (not illustrated) configured to receive the user's input, a communication circuit (not illustrated) configured to transmit the result values of analysis of biological constituent components to another device, and the like.

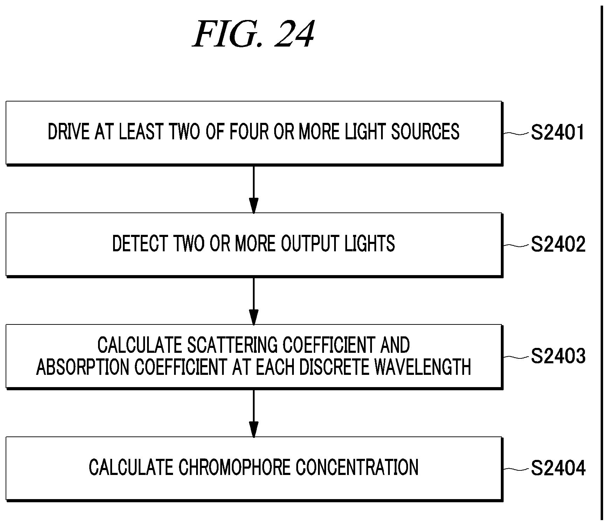

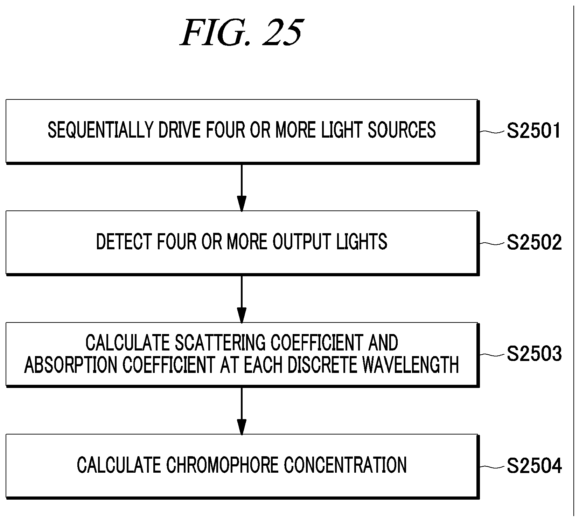

**FIG. 24** and **FIG. 25** are flowcharts each provided to explain a method of operating the bio-signal analyzing apparatus 10 according to an embodiment of the present disclosure. The method of operating the bio-signal analyzing apparatus 10 illustrated in **FIG. 24** and **FIG. 25** relates to the above-described embodiments illustrated in **FIG. 1** to **FIG. 23**. Therefore, the above descriptions with reference to **FIG. 1** to **FIG. 23** may be applied to the method of operating the bio-signal analyzing apparatus 10 illustrated in **FIG. 24** and **FIG. 25,** even though they are omitted hereinafter.

**FIG. 24** is a flowchart provided to explain a method of operating a bio-signal analyzing apparatus according to an embodiment of the present disclosure.

Referring to **FIG. 24,** the bio-signal analyzing apparatus 10 may drive at least two of four or more light sources configured to irradiate frequency-modulated light at four or more discrete wavelengths based on a chromophore present in a subject (S2401). Herein, the light sources may be LDs or LEDs capable of irradiating frequency-modulated light. In the following description, an example where the light sources are implemented as LDs will be described.

For example, the bio-signal analyzing apparatus 10 includes four or more LDs determined based on an already-known absorbance of a chromophore present in the body. Further, the four or more different discrete wavelengths may include a first discrete wavelength and a second discrete wavelength adjacent to peak regions in already-known absorption spectra of water ($H_2O$) and lipid, respectively, and a third discrete wavelength before an isosbestic point and a fourth discrete wavelength of a region adjacent to the isosbestic point in already-known absorption spectra of oxy-hemoglobin ($O_2Hb$) and deoxy-hemoglobin (HHb).

The bio-signal analyzing apparatus 10 may determine the number and kinds of LDs to be driven from among the four or more LDs based on at least one of the number, content, and kind of the chromophore present in the subject 20. For example, if the number of chromophores present in the subject 20 is four, the bio-signal analyzing apparatus 10 may determine at least four LDs that represent unique properties of the chromophores shown in the respective absorption spectra. However, even though the number of chromophores present in the subject 20 is four, if it is already known that any one chromophore is scarcely present (or present in a small content), the bio-signal analyzing apparatus 10 may determine at least three LDs having unique properties of the other three chromophores.

Further, the bio-signal analyzing apparatus 10 may sequentially drive the selected two or more LDs. For example, the bio-signal analyzing apparatus 10 may drive an LD that emits light at the shortest wavelength first and then drive LDs that emit light at a gradually longer wavelength, but the present disclosure may not be limited thereto.

Meanwhile, the bio-signal analyzing apparatus 10 may regulate the intensities of light output depending on at least one of mechanical properties and surrounding environments of the light sources and/or light detectors.

Then, the bio-signal analyzing apparatus 10 may detect two or more output lights reflected and introduced from the subject 20 when the two or more light sources are driven (S2402).

Then, the bio-signal analyzing apparatus 10 may calculate a scattering coefficient and an absorption coefficient at each discrete wavelength based on the two or more output lights (S2403). Firstly, the bio-signal analyzing apparatus 10 obtains a diffusion model. Then, the bio-signal analyzing apparatus 10 may calculate a scattering coefficient and a absorption coefficient of each output light by calculating an amplitude and phase of each output light and fitting the calculated amplitude and phase of the output light into the diffusion model. This process has been described in detail with reference to STEP 1 and STEP 2 of **FIG. 4.** Therefore, a detailed explanation thereof will be omitted.

Then, the bio-signal analyzing apparatus 10 may calculate a concentration of the chromophore present in the subject 20 based on the scattering coefficient and the absorption coefficient at each discrete wavelength (S2404). To be specific, the bio-signal analyzing apparatus 10 may calculate a concentration value of the chromophore present in the subject 20 by fitting (or calculating) with the scattering coefficient and the absorption coefficient at each discrete wavelength and an already-known extinction coefficient spectrum of the chromophore present in the subject 20. This process has been described in detail with reference to STEP 3 of **FIG. 4.** Therefore, a detailed explanation thereof will be omitted.

**FIG. 25** is a flowchart provided to explain a method of operating a bio-signal analyzing apparatus according to another embodiment of the present disclosure.

Referring to **FIG. 25,** the bio-signal analyzing apparatus 10 may sequentially drive four or more light sources configured to irradiate frequency-modulated light at four or more discrete wavelengths determined based on a chromophore present in the subject 20 (S2501).

Then, the bio-signal analyzing apparatus 10 may sequentially obtain four or more output lights reflected and introduced from the subject 20 when the four or more light sources are sequentially driven (S2502).

Then, the bio-signal analyzing apparatus 10 may calculate a scattering coefficient and an absorption coefficient at each discrete wavelength based on the four or more output lights (S2503).

Further, the bio-signal analyzing apparatus 10 may calculate a concentration of the chromophore present in the subject 20 based on the scattering coefficient and the absorption coefficient at each discrete wavelength (S2504). In this case, if a scattering coefficient and an absorption coefficient at a specific discrete wavelength are not significant data, the bio-signal analyzing apparatus 10 may exclude the scattering coefficient and the absorption coefficient for the discrete wavelength.

Meanwhile, the processes described in **FIG. 24** and **FIG. 25** may be divided into additional processes or combined into fewer processes depending on an embodiment of the present disclosure. In addition, some of the processes may be omitted and the sequence of the processes may be changed if necessary.

The embodiment of the present disclosure can be embodied in a storage medium including instruction codes executable by a computer such as a program module executed by the computer. A computer-readable medium can be any usable medium which can be accessed by the computer and includes all volatile/non-volatile and removable/non-removable media. Further, the computer-readable medium may include all computer storage media. The computer storage medium includes all volatile/non-volatile and removable/non-removable media embodied by a certain method or technology for storing information such as computer-readable instruction code, a data structure, a program module or other data.

The above description of the present disclosure is provided for the purpose of illustration, and it would be understood by a person with ordinary skill in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A frequency domain (FD)-based multi-wavelength bio-signal analyzing apparatus, comprising:

   four or more light sources configured to irradiate frequency-modulated light at four or more different discrete wavelengths;
   at least one light detector configured to detect an output light reflected and introduced from a subject; and
   a processing circuit connected to the four or more light sources and the at least one light detector and configured to calculate a scattering coefficient and an absorption coefficient at each discrete wavelength based on an output light detected by the at least one light detector and calculate a concentration of a chromophore present in the subject based on the scattering coefficient and the absorption coefficient at each discrete wavelength, wherein the processing circuit drives at least two of the four or more light sources based on the chromophore present in the subject.

2. The FD-based multi-wavelength bio-signal analyzing apparatus of Claim 1, wherein the processing circuit determines the number and kinds of light sources to be driven from among the four or more light sources based on at least one of the number, kind, and content of the chromophore present in the subject.

3. The FD-based multi-wavelength bio-signal analyzing apparatus of Claim 1, wherein the chromophore present in the subject includes at least one of oxy-hemoglobin (O2Hb), deoxy-hemoglobin (HHb), water (H2O), and lipid.

4. The FD-based multi-wavelength bio-signal analyzing apparatus of Claim 1, wherein the processing circuit calculates the scattering coefficient and the absorption coefficient at each discrete wavelength by fitting an amplitude and phase of the output light into a diffusion model, and calculate a concentration value of the chromophore present in the subject by fitting the scattering coefficient and the absorption coefficient at each discrete wavelength into an already-known extinction coefficient spectrum of the chromophore present in the subject.

5. The FD-based multi-wavelength bio-signal analyzing apparatus of Claim 1, wherein the processing circuit performs calibration to compensate for a phase and amplitude of a signal caused by mechanical properties from the output light.

6. The FD-based multi-wavelength bio-signal analyzing apparatus of Claim 1, wherein the four or more different discrete wavelengths are discontinuous wavelengths in a near infrared ray region.

7. The FD-based multi-wavelength bio-signal analyzing apparatus of Claim 1, wherein the four or more different discrete wavelengths are determined based on an already-known absorbance of a chromophore present in the body.

8. The FD-based multi-wavelength bio-signal analyzing apparatus of Claim 1, wherein the four or more different discrete wavelengths include a first discrete wavelength and a second discrete wavelength adjacent to peak regions in already-known absorption spectra of water (H2O) and lipid, respectively, and a third discrete wavelength before an isosbestic point and a fourth discrete wavelength of a region adjacent to the isosbestic point in already-known absorption spectra of oxy-hemoglobin (O2Hb) and deoxy-hemoglobin (HHb).

9.  The FD-based multi-wavelength bio-signal analyzing apparatus of Claim 1,
    wherein the four or more different discrete wavelengths include wavelengths of 688 nm, 808 nm, 915 nm, and 975 nm.

10. The FD-based multi-wavelength bio-signal analyzing apparatus of Claim 1,
    wherein the four or more light sources are implemented as laser diodes (LDs) or light emitting diodes (LEDs), respectively.

11. The FD-based multi-wavelength bio-signal analyzing apparatus of Claim 10,
    wherein the four or more LDs are multi-frequency vertical cavity surface emitting laser devices (VCSELs), respectively.

12. The FD-based multi-wavelength bio-signal analyzing apparatus of Claim 10,
    wherein each of the LDs is implemented as a vertical cavity surface emitting laser device (VCSEL),, and
    the four or more vertical cavity surface emitting laser devices are arranged spaced from each other on a contact surface in contact with the subject.

13. The FD-based multi-wavelength bio-signal analyzing apparatus of Claim 1,
    wherein the at least one light detector includes at least one avalanche photodiode (APD).

14. The FD-based multi-wavelength bio-signal analyzing apparatus of Claim 1,
    wherein the processing circuit sequentially drives the two or more light sources.

15. The FD-based multi-wavelength bio-signal analyzing apparatus of Claim 14,
    wherein the processing circuit drives a light source that emits light at the shortest wavelength first from among the two or more light sources and then drives light sources that emit light at a gradually longer wavelength.

16. The FD-based multi-wavelength bio-signal analyzing apparatus of Claim 1,
    wherein the processing circuit regulates the intensities of light output from the two or more light sources depending on at least one of mechanical properties and surrounding environments of the light sources.

17. The FD-based diffuse optical spectroscopic imaging apparatus of Claim 1, further comprising:

    a housing including the four or more light sources, the at least one light detector, and the processing circuit;
    a first optical fiber coupled to the four or more light sources and configured to concentrate light irradiated from the four or more light sources and transmit the light to the subject; and
    a second optical fiber coupled to at least one light detector and configured to concentrate light reflected from the subject and transmit the light to the at least one light detector,
    wherein the first optical fiber and the second optical fiber are exposed to the outside of the housing to be in contact with the subject.

18. The FD-based multi-wavelength bio-signal analyzing apparatus of Claim 1, further comprising:

    a probe including the four or more light sources, the at least one light detector, and a contact surface in contact with the subject,
    wherein the four or more light sources are arranged on the contact surface of the probe and directly irradiate light to the subject at the two or more different discrete wavelengths.

19. The FD-based multi-wavelength bio-signal analyzing apparatus of Claim 18,
    wherein the probe further includes a lens arranged on the contact surface of the probe and combined with the four or more light sources.

20. The FD-based multi-wavelength bio-signal analyzing apparatus of Claim 1,
    a probe including multiple light emission unit including the four or more light sources, multiple detection units including the at least one light detector, and a contact surface in contact with the subject,

wherein the multiple light emission units and the multiple detection units are arranged as crossed with each other on the contact surface of the probe.

21. The FD-based multi-wavelength bio-signal analyzing apparatus of Claim 20,
wherein the processing circuit sequentially drives the multiple light emission units, and
if one of the multiple light emission units is driven, the processing circuit drives at least one detection unit adjacent to the driven light emission unit to detect an output light.

22. The FD-based multi-wavelength bio-signal analyzing apparatus of Claim 20,
wherein the processing circuit sequentially drives the multiple light emission units, and
if one of the multiple light emission units is driven, the processing circuit drives two or more detection units arranged at different distances from the driven light emission unit to detect an output light.

23. The FD-based multi-wavelength bio-signal analyzing apparatus of Claim 20,
wherein the multiple light emission units and the multiple detection units are arranged at different distances as crossed with each other on the contact surface of the probe.

24. The FD-based multi-wavelength bio-signal analyzing apparatus of Claim 1,
wherein the FD-based multi-wavelength bio-signal analyzing apparatus includes four light sources, five light sources, six light sources, seven light sources, or eight light sources.

25. A frequency domain (FD)-based multi-wavelength bio-signal analyzing apparatus, comprising:

four or more light sources configured to irradiate frequency-modulated light at four or more different discrete wavelengths;
at least one light detector configured to detect an output light reflected and introduced from a subject;
a processing circuit connected to the four or more light sources and the at least one light detector and configured to calculate a scattering coefficient and an absorption coefficient at each discrete wavelength based on an output light detected by the at least one light detector and calculate a concentration of a chromophore present in the subject based on the scattering coefficient and the absorption coefficient at each discrete wavelength;
a first optical fiber coupled to the four or more light sources and configured to concentrate and transmit light irradiated from the four or more light sources;
a second optical fiber coupled to at least one light detector and configured to concentrate light reflected from the subject and transmit the light to the at least one light detector; and
a housing including the four or more light sources, the at least one light detector, and the processing circuit, wherein the first optical fiber and the second optical fiber are exposed to the outside of the housing to be in contact with the subject.

26. The FD-based multi-wavelength bio-signal analyzing apparatus of Claim 25,
wherein the processing circuit drives at least two of the four or more light sources based on at least one of the number, content, and kind of the chromophore present in the subject.

27. The FD-based multi-wavelength bio-signal analyzing apparatus of Claim 26,
wherein the processing circuit sequentially drives the at least two light sources.

28. A frequency domain (FD)-based multi-wavelength bio-signal analyzing apparatus, comprising:

at least one light emission unit in which four or more light sources configured to irradiate frequency-modulated light at four or more different discrete wavelengths are arranged spaced at a predetermined distance from each other;
at least one detection unit including a light detector configured to detect an output light reflected and introduced from a subject;
a probe including the at least one light emission unit and the at least one detection unit and having a contact surface in contact with the subject; and
a processing circuit connected to the at least one light emission unit and the at least one detection unit and configured to calculate a scattering coefficient and an absorption coefficient at each discrete wavelength based on an output light detected by the at least one detection unit and calculate a concentration of a chromophore present in the subject based on the scattering coefficient and the absorption coefficient,

wherein the at least one light emission unit and the at least one detection unit are arranged on the contact surface of the probe to be in direct contact with the subject.

29. The FD-based multi-wavelength bio-signal analyzing apparatus of Claim 28,
wherein the processing circuit drives at least two of the four or more light sources included in each light emission unit based on at least one of the number, content, and kind of the chromophore present in the subject.

30. The FD-based multi-wavelength bio-signal analyzing apparatus of Claim 29,
wherein the processing circuit sequentially drives the at least two light sources.

31. The FD-based multi-wavelength bio-signal analyzing apparatus of Claim 28,
wherein the four or more light sources are multi-frequency vertical cavity surface emitting laser devices (VCSELs), respectively

32. The FD-based multi-wavelength bio-signal analyzing apparatus of Claim 28,
wherein each of the light sources is implemented as a vertical cavity surface emitting laser device (VCSEL), and the four or more vertical cavity surface emitting laser devices are arranged spaced from each other on the contact surface.

33. The FD-based multi-wavelength bio-signal analyzing apparatus of Claim 28,
wherein the FD-based multi-wavelength bio-signal analyzing apparatus includes multiple light emission units and multiple detection units, and
the multiple light emission units and the multiple detection units are arranged as crossed with each other on the contact surface of the probe.

34. The FD-based multi-wavelength bio-signal analyzing apparatus of Claim 33,
wherein the processing circuit sequentially drives the multiple light emission units, and
if one of the multiple light emission units is driven, the processing circuit drives at least one detection unit adjacent to the driven light emission unit to detect an output light.

35. The FD-based multi-wavelength bio-signal analyzing apparatus of Claim 33,
wherein the processing circuit sequentially drives the multiple light emission units, and
if one of the multiple light emission units is driven, the processing circuit drives two or more detection units arranged at different distances from the driven light emission unit to detect an output light.

36. The FD-based multi-wavelength bio-signal analyzing apparatus of Claim 28,
wherein the processing circuit is implemented as included in the probe.

37. A method of operating an FD-based multi-wavelength bio-signal analyzing apparatus, comprising:

driving at least two of four light sources configured to irradiate frequency-modulated light at four or more different discrete wavelengths based on a chromophore present in a subject;
detecting two or more output lights reflected and introduced from the subject when the two or more light sources are driven;
calculating a scattering coefficient and an absorption coefficient at each discrete wavelength based on the two or more output lights; and
calculating a concentration of the chromophore present in the subject based on the scattering coefficient and the absorption coefficient at each discrete wavelength.

38. The method of Claim 37,
wherein the driving of the at least two of four light sources includes determining the number and kinds of light sources to be driven from among the four or more light sources based on at least one of the number, content, and kind of the chromophore present in the subject.

39. The method of Claim 37,
wherein the calculating of the scattering coefficient and the absorption coefficient at each discrete wavelength includes:

calculating an amplitude and phase of each output light; and
calculating the scattering coefficient and the absorption coefficient at each discrete wavelength by fitting the amplitude and phase of the output light into a diffusion model.

40. The method of Claim 39,
Wherein the calculating of the amplitude and phase of the output light includes compensating for a phase and amplitude of a signal caused by mechanical properties from the output light.

41. The method of Claim 37,
wherein the calculating of the concentration of the chromophore present in the subject includes calculating the concentration of the chromophore present in the subject by fitting the scattering coefficient and the absorption coefficient at each discrete wavelength into an already-known extinction coefficient spectrum of the chromophore present in the subject.

42. The method of Claim 37,
wherein the four or more different discrete wavelengths are determined based on an already-known absorbance of a chromophore present in the body.

43. The method of Claim 37,
wherein the four or more different discrete wavelengths include a first discrete wavelength and a second discrete wavelength adjacent to peak regions in already-known absorption spectra of water ($H_2O$) and lipid, respectively, and a third discrete wavelength before an isosbestic point and a fourth discrete wavelength of a region adjacent to the isosbestic point in already-known absorption spectra of oxy-hemoglobin ($O_2Hb$) and deoxy-hemoglobin (HHb).

44. The method of Claim 37,
wherein the driving of the two or more light sources includes driving a light source that emits light at the lowest wavelength first from among the two or more light sources and then driving light sources that emit light at a gradually higher wavelength.

45. The method of Claim 37,
wherein the driving of the two or more light sources includes regulating the intensities of light output from the two or more light sources depending on at least one of mechanical properties and surrounding environments.

46. A frequency domain (FD)-based multi-wavelength bio-signal analyzing apparatus, comprising:

four or more laser diodes (LDs) configured to irradiate frequency-modulated light at four or more different discrete wavelengths determined based on a chromophore present in a subject;
at least one light detector configured to detect an output light emitted from the subject; and
a processing circuit connected to the four or more LDs and the at least one light detector and configured to sequentially drive the four or more LDs, obtain four or more output lights detected by the at least one light detector when the four or more LDs are sequentially driven, calculate a scattering coefficient and an absorption coefficient at each discrete wavelength based on the four or more output lights, and calculate a concentration of the chromophore present in the subject based on the scattering coefficient and the absorption coefficient at each discrete wavelength.

47. A method of operating a frequency domain (FD)-based multi-wavelength bio-signal analyzing apparatus, comprising:

sequentially driving four or more laser diodes (LDs) configured to irradiate frequency-modulated light at four or more different discrete wavelengths determined based on a chromophore present in a subject;
sequentially detecting four or more output lights emitted form the subject when the four or more LDs are sequentially driven;
calculating a scattering coefficient and an absorption coefficient at each discrete wavelength based on the four or more output lights; and
calculating a concentration of the chromophore present in the subject based on the scattering coefficient and the absorption coefficient at each discrete wavelength.

48. A computer-readable storage medium in which a program configured to implement a method of any one of Claims 37 is stored.

# FIG. 1

# FIG. 2

## FIG. 3

INPUT LIGHT
(L_In)

REFLECTED LIGHT
(L_Out)

20

intensity

301

INPUT LIGHT(L_In)

302

REFLECTED
LIGHT
(L_Out)

time

300

# FIG. 4

STEP 2-1: PERFORM CALIBRATION

STEP 1: CALCULATE DIFFUSE MODEL

STEP 2: CALCULATE SCATTERING COEFFICIENT AND ABSORPTION COEFFICIENT OF OUTPUT LIGHT

STEP 3: CALCULATE CONCENTRATION OF CHROMOPHORE BASED ON SCATTERING COEFFICIENTS AND ABSORPTION COEFFICIENTS OF MULTIPLE OUTPUT LIGHTS

## FIG. 5

EP 3 460 453 A2

## FIG. 6

EP 3 460 453 A2

## FIG. 7

FIG. 8

## FIG. 9

900

EP 3 460 453 A2

# FIG. 10

Water Concentration With Broadband Fit 95% CI

EP 3 460 453 A2

*FIG. 11*

Lipid Concentration With Broadband Fit 95% CI

*FIG. 12*

Oxy-Hemoglobin Concentration With Broadband Fit 95% CI

EP 3 460 453 A2

## FIG. 13

Deoxy-Hemoglobin Concentration With Broadband Fit 95% CI

## FIG. 14

# FIG. 15

## FIG. 16

*FIG. 17*

# FIG. 18

## FIG. 19

## FIG. 20

10e

2030
Processing
Circuit

2010
LIGHT
EMISSION UNIT

2020
DETECTION
UNIT

2001

2002

20

# FIG. 21

2002

S1 – S8 : LIGHT EMISSION UNIT
D1 – D8 : DETECTION UNIT

*FIG. 22*

# FIG. 23

2002

D1  S1  D2  S2

S3  D3

S4  D4

# FIG. 24

DRIVE AT LEAST TWO OF FOUR OR MORE LIGHT SOURCES — S2401

DETECT TWO OR MORE OUTPUT LIGHTS — S2402

CALCULATE SCATTERING COEFFICIENT AND ABSORPTION COEFFICIENT AT EACH DISCRETE WAVELENGTH — S2403

CALCULATE CHROMOPHORE CONCENTRATION — S2404

# FIG. 25

| | |
|---|---|
| SEQUENTIALLY DRIVE FOUR OR MORE LIGHT SOURCES | ~S2501 |

↓

| | |
|---|---|
| DETECT FOUR OR MORE OUTPUT LIGHTS | ~S2502 |

↓

| | |
|---|---|
| CALCULATE SCATTERING COEFFICIENT AND ABSORPTION COEFFICIENT AT EACH DISCRETE WAVELENGTH | ~S2503 |

↓

| | |
|---|---|
| CALCULATE CHROMOPHORE CONCENTRATION | ~S2504 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7428434 B